# EUROPÄISCHE PATENTSCHRIFT

(11) **EP 2 566 946 B1**
(45) Veröffentlichungstag und Bekanntmachung des Hinweises auf die Patenterteilung: **09.04.2014**
(21) Anmeldenummer: 11719230.2
(22) Anmeldetag: 06.05.2011
(51) Int. Cl.: C12M 1/107, C12P 5/02

(54) **VERFAHREN UND ANLAGE ZUR GASDICHTEN PROZESSFÜHRUNG VON PERKOLATOREN IN EINEM ZWEI- ODER MEHRSTUFIGEN BIOGASVERFAHREN**
METHOD AND SYSTEM FOR THE GAS TIGHT PROCESS CONTROL OF PERCOLATORS IN A BIOGAS METHOD HAVING TWO OR MORE STAGES
PROCÉDÉ ET INSTALLATION D'EXÉCUTION DE TRAITEMENT ÉTANCHE AUX GAZ PAR DES APPAREILS DE PERCOLATION DANS UN PROCÉDÉ DE PRODUCTION DE BIOGAZ EN DEUX ÉTAPES OU PLUS

(30) Priorität: 06.05.2010 DE 102010028707
(43) Veröffentlichungstag der Anmeldung: 13.03.2013
(73) Patentinhaber: Gicon Grossmann Ingenieur Consult GmbH, 01219 Dresden (DE)
(72) Erfinder: GROSSMANN, Jochen, 01187 Dresden (DE)
(74) Vertreter: Uhlemann, Henry
(86) Internationale Anmeldenummer: PCT/EP2011/057282
(87) Internationale Veröffentlichungsnummer: WO 2011/138426

(56) Entgegenhaltungen:
- EP-A2- 2 103 681
- WO-A1-2007/012328
- WO-A2-2010/028643

## Beschreibung

Die Erfindung betrifft ein Verfahren und eine Anlage zur Perkolation von festen biogenen Stoffen in einem zwei- oder mehrstufigen Biogasverfahren. Die Erfindung findet Anwendung in der regenerativen Energieerzeugung.

Die Gewinnung von Biogas aus nachwachsenden Rohstoffen, bioverfügbaren Abfällen und sonstigen Einsatzstoffen erfolgt mit Hilfe von Biogasanlagen, in denen Mikroorganismen eine biochemische Umwandlung dieser Stoffe in Biogas, bestehend aus den Hauptkomponenten Methan und Kohlendioxid, vornehmen.

Die Umwandlung von biologisch abbaubaren Stoffen (im Folgenden als biogene Stoffe bezeichnet) in Biogas erfolgt über mehrere biochemische Schritte, die Hydrolyse, Acidogenese, Acetogenese und Methanogenese.

In der Hydrolyse werden wasserlösliche Bestandteile aus den biogenen Stoffen herausgelöst sowie durch mehrere Arten extrazellulärer Enzyme nicht wasserlösliche biogene Stoffe in wasserlösliche, in der Regel niedermolekulare, Stoffe zersetzt. Zur Beschleunigung bestimmter Abbauvorgänge können auch sogenannte Fremdenzyme eingesetzt werden. In der folgenden Acidogenese werden die in der Hydrolyse gelösten Stoffe in niedere organische Säuren, wie niedere Fettsäuren und Aminosäuren, umgewandelt. In der Acetogenese werden die organischen Säuren in Essigsäure unter Bildung von CO₂ umgewandelt. Die Produkte der Acetogenese werden in der Methanogenese mit Hilfe von methanbildenden Bakterien zu Methan umgewandelt.

Diese Prozesse finden in einstufigen Biogasanlagen zeitlich und räumlich nebeneinander statt. In zweistufigen Biogasverfahren werden die Teilschritte Hydrolyse und Acidogenese (erste Stufe) von den Teilschritten Acetogenese und Methanogenese (zweite Stufe) apparate- und prozesstechnisch getrennt. Dadurch ist es möglich, die unterschiedlichen Milieubedingungen für die während der Hydrolyse und der Methanisierung ablaufenden Umwandlungsprozesse getrennt zu regeln. So werden eine bessere Steuerbarkeit und eine höhere Stabilität des Verfahrens erreicht. Dadurch können in zwei- und mehrstufigen Biogasanlagen im Gegensatz zu einstufigen Biogasanlagen höhere Methankonzentrationen im Biogas erreicht werden. Durch die prozesstechnische Abtrennung der Hydrolyse können unterschiedliche Substrate umgewandelt werden, so dass ein modularer Aufbau der Biogasanlage möglich ist.

Im üblichen Sprachgebrauch wird die erste Stufe des zweistufigen Biogasverfahrens häufig nur als Hydrolysestufe und die zweite Stufe als Methanstufe bezeichnet. Die Hydrolysestufe findet im sogenannten Hydrolysereaktor statt. Die Methanisierung erfolgt im sogenannten Methanreaktor. Die die Hydrolyse verlassende wässrige Lösung wird gemeinhin als Hydrolysat bezeichnet. Im Folgenden wird diese vereinfachende Sprachweise ebenfalls genutzt.

Im Hydrolysereaktor erfolgt der Abbau der biogenen Stoffe zu niederen organischen Säuren unter Bildung eines Hydrolysegases. Das Hydrolysegas wird üblicherweise aus dem Prozess abgeführt, ohne innerhalb des Prozesses weiter genutzt zu werden.

Zur Hydrolyse fester biogener Stoffe sind verschiedene Verfahren geeignet. Neben der Hydrolyse in Rührbehältern oder Propfenstromfermentern finden Verfahren der Perkolation verbreitet Anwendung. Bei der Perkolation werden die festen biogenen Stoffe in Hydrolysereaktoren, sogenannten Perkolatoren, gestapelt und mit Flüssigkeit (Prozesswasser) berieselt. Die bei der Perkolation gebildete, mit organischen Säuren beladene Flüssigkeit (Hydrolysat, hierin auch als Perkolat bezeichnet) wird aus den Perkolatoren zu Zwischenspeichern transportiert. Das in einem entsprechenden Hydrolysatbehälter gespeicherte Hydrolysat wird geregelt zum Methanreaktor zugeführt. In diesem Reaktor erzeugen anaerob lebende methanbildende Mikroorganismen das methanhaltige Biogas. Mit dieser Dosierungsregelung wird eine Steuerung der Methanbildung möglich. Die Methanbildung erfolgt nach gegenwärtigem Stand des Wissens auf zwei Wegen, der acetotrophen und der hydrogenotrophen Umwandlung, die im Methanreaktor parallel ablaufen. Die in der Hydrolysatflüssigkeit enthaltenen organischen Stoffe werden dabei zu Methan und weiteren Nebenprodukten umgewandelt. Die verbleibende, von organischen Abbauprodukten der biogenen Stoffe weitgehend befreite Flüssigkeit wird als Gärflüssigkeit bezeichnet. Die Gärflüssigkeit wird aus dem Methanreaktor abgeführt.

Die Hydrolyse von festen biogenen Stoffen ist aus dem Stand der Technik bekannt und wird mit Hilfe einer sogenannten aeroben Perkolation (hierin auch "offene Perkolation" oder "offene Hydrolyse") durchgeführt. Im Gegensatz zu anaeroben Verfahren, welche zwingend über gasdicht ausgeführte Perkolatoren verfügen müssen, besteht bei der aeroben Perkolation die Möglichkeit des Luftzutritts und der aeroben Umsetzung von organischen Bestandteilen zu Kohlendioxid und Wasser, sowie der unmittelbaren Gasabführung zur Atmosphäre, so dass Verluste in Bezug auf das Biogasbildungspotential und ein kontinuierliches Entweichen von gebildetem Hydrolysegas erfolgen. Hydrolysegas enthält bei aerober Betriebsweise vor allem Kohlendioxid und in geringen Mengen auch Wasserstoff, Methan und in Spuren andere Gase, z.B. H₂S, enthalten kann.

WO 2006/048008 und WO 2007/012328 A1 beschreiben jeweils zweistufige Biogasverfahren, bei denen eine aerobe Perkolation durchgeführt wird, so dass das gebildete Hydrolysegas in die Atmosphäre entweichen kann. Der aerobe Umsatz der biogenen Stoffe führt zur vermehrten Bildung von Kohlendioxid und Wasser, so dass dadurch nachteilig der nutzbare Energieinhalt des Substrats reduziert wird.

Zusätzlich zum durch die Methanisierung gebildeten Biogas kann auch das bei der Perkolation gebildete Hydrolysegas anteilig Methan enthalten. Dies tritt insbesondere dann auf, wenn die Sauerstoffzufuhr zur Perkolation eingeschränkt oder unterbunden wird.

Bei einer offenen Perkolation kann ggf. gebildetes Methan in die Atmosphäre entweichen. Dies ist sowohl für die ökonomische als auch für die ökologische Erzeugung von Biogas nachteilig. Es bedeutet eine zusätzliche Belastung der Atmosphäre mit Treibhausgasen, sowie eine Minderung des Energiegewinns, da die entsprechenden Methanmengen einer energetischen Nutzung nicht mehr zur Verfügung stehen. Weiter wird durch den Eintrag von Sauerstoff bei der aeroben Perkolation der unter Energieverlust für den Biogasprozess stattfindende aerobe Abbau der biogenen Stoffe zur Kohlendioxid und Wasser begünstigt.

Wird die Perkolation gasdicht durchgeführt und so die Sauerstoffzufuhr in die Perkolatoren beschränkt oder völlig unterbunden, findet eine anaerobe Umsetzung der organischen Bestandteile statt. Zweistufige Biogasverfahren, bei denen eine anaerobe Perkolation vorgenommen wird, sind beispielsweise aus DE 10 2006 009 165 A1 bekannt. DE 10 2006 009 165 A1 offenbart ein Verfahren zur zweistufigen Gewinnung von Biogas aus Organik enthaltenden Abfallstoffen und einen zur Durchführung dieses Verfahrens geeigneten Reaktor. Der Perkolator wird nicht belüftet, so dass eine ausschließlich anaerobe Verfahrensführung der Hydrolyse vollzogen wird. Das dabei gebildete Hydrolysegas wird ungenutzt aus den Perkolatoren abgeleitet.

Durch die anaerobe Umsetzung der eingesetzten Organik kann die Methankonzentration im Hydrolysegas bei anaerober Perkolation höhere Werte erreichen, als bei aerober Perkolation. Um in aeroben Perkolationsverfahren bereits in der Hydrolysestufe energetisch nutzbares Methan zu erzeugen, sind Verfahren bekannt, bei denen methanbildende Mikroorganismen in die Hydrolysestufe durch Inokulation eingebracht werden.

Weiterhin kann der Umsatz zu Methan durch eine Erhöhung der Verweilzeit der Hydrolysatflüssigkeit in der Hydrolysestufe erfolgen. Dazu offenbart DE 10 2008 007 423 A1 ein zweistufiges Biogasverfahren und eine entsprechende Anlage, wobei zumindest ein Teil des Hydrolysegases in thermische Energie umgesetzt wird. Die aus dem Hydrolysegas gewonnene thermische Energie wird zur teilweisen Deckung des Energiebedarfs innerhalb der Biogasanlage genutzt. Die thermische Nutzung des Hydrolysegases ist jedoch unvorteilhaft, wenn der Methangehalt des Hydrolysegases gering, sein CO₂-Anteil jedoch hoch ist, da dann inertes Gas unter Energieaufwand transportiert werden muss.

Die erhöhte Methankonzentration im Perkolator kann bei erneuter Sauerstoffzufuhr zur Bildung zündfähiger Gasgemische führen. Dieser sicherheitsrelevante Zustand muss beim Betrieb von Perkolatoren beachtet werden. Liegt im gasdicht ausgeführten Perkolator Hydrolysegas mit einer Methankonzentration vor, die sicherheitstechnisch bedenklich ist, ist eine sichere Abführung des Hydrolysegases erforderlich. Zudem sollte die Methankonzentration des Gases im Perkolator speziell vor dem Entleeren der Perkolatoren so weit reduziert werden, dass eine zündfähige Atmosphäre bei Öffnen der Behälter ausgeschlossen werden kann.

Daher wird das im Perkolator enthaltene Gas (hierin auch als "Gasatmosphäre" des Perkolators bezeichnet) vor dem Öffnen des Perkolators üblicherweise durch Verbrennung über Gasfackeln entsorgt. Dafür ist meist die Nutzung einer weiteren Energiequelle in Form einer Zufeuerung notwendig, da die alleinige Verfeuerung des Gases zumeist nicht möglich ist.

Damit ein Entweichen von sicherheitskritischen Konzentrationen von Methan aus den gasdicht ausgestalteten Perkolatoren verhindert wird, sind Lösungen bekannt, um die Methankonzentration im Hydrolysegas zu reduzieren.

EP 1 301 583 B1 offenbart eine Biogasanlage zur einstufigen Methanisierung durch Trockenfermentation, welche sich durch eine erhöhte Sicherheit auszeichnet. Dafür ist die Anlage mit einem Sensor versehen, der den Sauerstoffpartialdruck im Fermenter misst. Überschreitet der Sauerstoffpartialdruck einen bestimmten Grenzwert, ist dies ein Anzeichen für das Eindringen von Sauerstoff durch ein Leck. Automatisch wird die Biogasleitung verschlossen und Abgas aus einem Biogasverbraucher zugeleitet, welches im Wesentlichen aus Kohlendioxid besteht. Durch ein Spülventil können die im Fermenter befindlichen Gase entweichen, so dass zum Schluss nahezu ausschließlich Kohlendioxid im Behälter verbleibt.

In EP 2 103 681 A2 ist als Weiterbildung der Anlage aus EP 1 301 583 B1 ist eine Lösung offenbart, in der zur Verdrängung methanhaltigen Biogases aus einem einstufigen Trockenfermentationsprozess kohlendioxidhaltiges Abgas aus einem Blockheizkraftwerk (BHKW) eingesetzt wird. Dadurch kann innerhalb einer einzigen Biogasanlage sowohl die Vergärung (anaerober Umsatz der festen biogenen Stoffe zu Biogas aus Methan und Kohlendioxid) als auch die Kompostierung des zuvor vergorenen Substrats (aerober Prozess) erfolgen, ohne dass das Substrat zur Kompostierung umgebettet werden muss. Das Verfahren ist derartig gestaltet, dass in einem Prozess der einstufigen Biogasgewinnung zum Ende der Vergärung eine Spülung der Gasphase des Fermenters erfolgt. Dafür wird zum Ende der Vergärung kohlendioxid-haltiges Abgas aus einem BHKW zugeleitet. Die Methankonzentration des im Fermenter enthaltenen Gases wird mit Hilfe eines Sensors ermittelt. Liegt der Methangehalt des Gases oberhalb eines bestimmten Grenzwertes (bei dem die energetische Nutzung des Gases sinnvoll ist), wird das Gas dem BHKW zugeführt. Wird der Grenzwert unterschritten, wird das Gas zu einer Abgasfackel zur Verbrennung geleitet. Ggf. wird dabei zusätzlicher Brennstoff zugeführt. Sinkt der Methangehalt des Gases weiter unter einen zweiten, niedrigeren Grenzwert (bei dem ein gefahrloses Ableiten des Gases aus dem Fermenter möglich ist), wird anstelle von kohlendioxid-haltigem Abgas Frischluft in den Fermenter geleitet und gleichzeitig das Gasgemisch über einen Bioabgaskamin in die Umgebung freigegeben. Durch die Frischluftzufuhr kann so auch der Kompostierungsprozess in der Anlage vorgenommen werden.

Die in EP 1 301 583 B1 und EP 2 103 681 A2 offenbarten Anlagen und Verfahren stellen einstufige Biogasgewinnungsprozesse dar, die den Nachteil haben, dass die erzielte Methankonzentration im Biogas limitiert ist. Desweiteren muss insbesondere bei der in EP 2 103 681 A2 offengelegten Verfahrensweise Energie aufgewendet werden, um CO₂-reiche BHKW-Abgase zu den Reaktoren zurückzuführen. Dies senkt den Gesamtwirkungsgrad der Energieerzeugung einer solchen Anlage.

Aufgabe der Erfindung ist es, ein Verfahren und eine Anlage zur zweistufigen Biogasgewinnung bereitzustellen, bei dem die bei der Hydrolyse gebildeten Gase besser genutzt werden.

Die Aufgabe wird erfindungsgemäß gelöst durch ein Verfahren zur zwei- oder mehrstufigen Biogasgewinnung durch Hydrolyse fester biogener Stoffe in mindestens zwei zeitlich versetzt betriebenen Perkolatoren, welches eine Hydrolyse- und eine Methanstufe umfasst. Dabei werden die biogenen Stoffe in der Hydrolysestufe hydrolysiert, wobei im Perkolator flüssiges Hydrolysat und Hydrolysegas entsteht. Dabei entsteht CO₂-reiches Hydrolysegas und anschließend methanhaltiges Hydrolysegas. Das Hydrolysat wird aus den Perkolatoren entnommen und gesammelt, wobei ein Teil des Hydrolysats der Methanstufe zugeführt wird und der andere Teil des Hydrolysats der Hydrolysestufe zugeführt wird. In der Methanstufe wird das Hydrolysat durch methanbildende Mikroorganismen zu Biogas und Gärflüssigkeit umgewandelt. Die Gärflüssigkeit wird aus dem Methanreaktor entnommen und gesammelt, und ggf. der Hydrolysestufe zugeführt. Nach dem erfindungsgemäßen Verfahren werden die Perkolatoren gasdicht betrieben und Hydrolysegas wird aus den Perkolatoren abgeleitet. Dabei wird das methanhaltige Hydrolysegas einer energetischen Nutzung zugeführt und CO₂-reiches Hydrolysegas aus einem Perkolator wird zur Spülung von einem weiteren, zu dem zu spülenden Perkolator zeitlich versetzt betriebenen Perkolator genutzt.

Die Erfindung beruht auf der Beobachtung, dass während der Perkolation fester biogener Stoffe besonders gegen Ende des Perkolationsvorgangs vermehrt Methan gebildet wird, jedoch zu Beginn des Perkolationsvorgangs Hydrolyse-Gas mit einem hohen CO₂-Anteil entsteht.

Dies geschieht im erfindungsgemäßen Verfahren durch das Wachstum und die Entwicklung der autochthon im Substrat vorhandenen Mikroorganismen und dadurch, dass Hydrolysat und Gärflüssigkeit der Hydrolysestufe zugeführt werden (diese Prozessführung der Flüssigkeit wird hierin auch als "Kreislaufbetrieb" der jeweiligen Flüssigkeit bezeichnet). Die Zuführung von Hydrolysat und Gärflüssigkeit zur Hydrolysestufe erfolgt dabei aus Speicherbehältern, welche jeweils der Hydrolysestufe (Hydrolysat-Speicherbehälter) oder der Methanstufe (Gärflüssigkeits-Speicherbehälter) nachgeordnet sind.

Dadurch werden methanbildende Mikroorganismen aus der Methanstufe in die Hydrolysestufe eingetragen, die unter Sauerstoffabschluss im gasdichten Perkolator der Hydrolysestufe organische Bestandteile des Hydrolysats zu Methan umwandeln. Eine zusätzliche Inokulation der Hydrolysestufe mit methanbildenden Mikroorganismen wird im erfindungsgemäßen Verfahren nicht vorgenommen. Die im Perkolator vorhandenen methanbildenden Mikroorganismen sind im Substrat vorhanden oder werden durch die zugeführte Gärflüssigkeit eingetragen. Im erfindungsgemäßen Verfahren wird das Hydrolysat direkt aus der Hydrolysestufe abgeführt. Es werden vorzugsweise keine Verfahrensmaßnahmen ergriffen, die die Verweilzeit des Hydrolysats in der Hydrolysestufe erhöhen, wodurch in der Hydrolysestufe die Umwandlung der Bestandteile des Hydrolysats zu Methan gefördert würde. Das im erfindungsgemäßen Verfahren in der Hydrolysestufe gebildete Methan wird prozessbedingt als Nebenprodukt der Hydrolyse gebildet.

Die Prozessbedingungen des erfindungsgemäßen Verfahrens zielen auf einen intensiven Stofftransport von wasserlöslichen organischen Verbindungen, die durch mikrobiologische und enzymatische Umwandlungsprozesse aus den eingesetzten festen biogenen Stoffen in die Hydrolysat-Flüssigkeit. Dies wird vorzugsweise durch eine saure Fahrweise (pH-Wert des Hydrolysats im sauren Bereich) realisiert. Bevorzugt wird dabei unter geregelter leichter Sauerstoffzufuhr (leicht aerob) gearbeitet.

Das erfindungsgemäße Verfahren ist ein zwei- oder mehrstufiges Verfahren zur Biogasgewinnung, welches eine Hydrolysestufe und eine Methanstufe umfasst, wobei lediglich in der Hydrolysestufe feste biogene Stoffe vorliegen. In der Hydrolysestufe werden die biogenen Stoffe durch mikrobiologische und enzymatische Umwandlungsprozesse zersetzt und wasserlösliche organische Bestandteile aus den biogenen Stoffen herausgelöst. Diese bilden mit der zur Hydrolyse eingetragenen wässrigen Flüssigkeit das Hydrolysat. In der Methanstufe der erfindungsgemäßen Verfahren werden organische Bestandteile ausschließlich durch das Hydrolysat, also durch eine wässrige Flüssigkeit, eingetragen. In der Methanstufe liegen im erfindungsgemäßen Verfahren bevorzugt keine biogenen Feststoffe vor.

Wird das erfindungsgemäße Verfahren mit mehreren Perkolatoren durchgeführt, sind diese bezüglich der Hydrolysatführung entweder parallel oder in Reihe geschaltet. Unter Parallelschaltung von Perkolatoren im Sinne der Erfindung wird verstanden, dass das aus den einzelnen Perkolatoren abgeführte Hydrolysat vor der Zuführung zur Methanstufe vereinigt wird. Unter Reihenschaltung von Perkolatoren im Sinne der Erfindung wird verstanden, dass das aus einem Perkolator abgeführte Hydrolysat zur Flüssigkeits-Zuleitung des jeweils nächsten Perkolators, welcher vorzugsweise zu einem früheren Zeitpunkt mit biogenen Stoffen beschickt wurde, geleitet wird.

Der Anteil der Inhaltsstoffe (insbesondere CO₂ und Methan) am Hydrolysegas ist abhängig von den eingesetzten festen biogenen Stoffen (Substrat).

Das unter anaeroben Bedingungen am Anfang des Perkolationsprozesses gebildete Hydrolysegas ist CO₂-reich und zeichnet sich weiter durch einen geringen Methananteil aus. Diese Fraktion des Hydrolysegases wird hierin auch als CO₂-reiches Hydrolysegas bezeichnet. CO₂-reiches Hydrolysegas weist vorzugsweise einen CO₂-Anteil von mindestens 50 Vol.-%, bevorzugt mindestens 70 Vol.-%, und einen Methananteil von weniger als 2 Vol.-%, vorzugsweise weniger als 0,5 Vol.-% auf. Der pH-Wert des Hydrolysats liegt am Anfang des Perkolationsprozesses im leicht sauren Bereich, vorzugsweise bei pH 4 bis pH 5. Eine energetische Nutzung des Hydrolysegases ist zu diesem Prozesszeitpunkt aufgrund des geringen Methananteils nicht sinnvoll. Aufgrund des hohen CO₂-Anteils ist diese Fraktion des Hydrolysegases zur Spülung anderer Perkolatoren des Verfahrens geeignet.

Mit fortschreitender Umwandlung der biogenen Stoffe während der anaeroben Perkolation steigt der Methananteil des Hydrolysegases und der CO₂-Anteil des Hydrolysegases sinkt. Diese Fraktion des Hydrolysegases wird hierin auch als methanhaltiges Hydrolysegas bezeichnet. Methanhaltiges Hydrolysegas weist vorzugsweise einen CO₂-Anteil von weniger als 70 Vol.-% und einen Methananteil von mindestens 2 Vol.-%, bevorzugt mindestens 8 Vol.-% auf. Gleichzeitig zum Ansteigen des Methananteils im Hydrolysegas steigt der pH-Wert des Hydrolysats in den leicht sauren bis neutralen Bereich, vorzugsweise liegt der pH-Wert zu diesem Zeitpunkt des Perkolationsprozesses bei pH > 5. Zu diesem Prozesszeitpunkt wird das methanhaltige Hydrolysegas mit Hilfe eines erfindungsgemäßen Verfahrens entnommen und einer energetischen Nutzung zugeführt.

Zur energetischen Nutzung wird das methanhaltige Hydrolysegas ggf. gereinigt und aufbereitet und in einem für die energetische Nutzung von Biogas üblichen Prozess genutzt, beispielsweise als Heizgas in Blockheizkraftwerken, zur Einspeisung in das Erdgasnetz oder zum Betrieb von Motoren durch Verbrennung. Eine Aufbereitung erfolgt dabei vorzugsweise durch aus dem Stand der Technik bekannte Verfahren der Gaswäsche und/oder Druckwechseladsorption. Dadurch kann der Anteil an Begleitgasen, vorzugsweise der Anteil von CO₂ und/oder der Anteil von H₂S, am methanhaltigen Hydrolysegas vermindert werden.

Alternativ zur direkten energetischen Nutzung wird das methanhaltige Hydrolysegas vor der energetischen Nutzung in den Methanreaktor eingeleitet oder mit dem im Methanreaktor gebildeten Biogas vereinigt. Dadurch können große Schwankungen des Methananteils des Biogases verhindert werden.

Um das methanhaltige Hydrolysegas einer energetischen Nutzung zuzuführen, wird die Perkolation im erfindungsgemäßen Verfahren gasdicht ausgestaltet, d.h. dass die Perkolatoren geschlossen sind und mit Gasleitungen versehen sind, die z.B. über Ventile oder Gasklappen eine kontrollierte Zu- und Ableitung von Gas ermöglichen.

Die Perkolation der festen biogenen Stoffe mit einem erfindungsgemäßen Verfahren umfasst innerhalb eines Perkolators vorzugsweise die zeitlich wie folgt angeordneten Betriebsstufen:
a. Beladung eines Perkolators mit den biogenen Stoffen (hierin auch als Beschickung eines Perkolators bezeichnet),
b. Abluftbetrieb,
c. Gasnutzungsbetrieb,
d. Spülung des Perkolators mit Abluft aus einem anderen, zeitlich versetzt betriebenen Perkolator,
e. Spülung des Perkolators mit Luft,
f. Öffnung des Perkolators.

Im Abluftbetrieb wird CO₂-reiches Hydrolysegas als Abluft aus dem Perkolator geleitet. Der Abluftbetrieb wird zum Anfang der Perkolation durchgeführt, solange das gebildete Hydrolysegas einen hohen CO₂-Anteil aufweist. Daher wird der Abluftbetrieb vorzugsweise so lange durchgeführt, bis die Methankonzentration des im Perkolator befindlichen Gases einen zuvor definierten Grenzwert erreicht und/oder bis der pH-Wert des Hydrolysats einen zuvor definierten Grenzwert erreicht. Der Grenzwert für die Methankonzentration ist substratabhängig und beträgt vorzugsweise mindestens 1 Vol.-% Methan, besonders bevorzugt mindestens 2 Vol.-% Methan. Eine energetische Nutzung des Hydrolysegases ist vorzugsweise dann sinnvoll, wenn der Methangehalt des Hydrolysegases mindestens 8 Vol.-% beträgt. Der Grenzwert für den pH-Wert des Hydrolysats ist ebenfalls substratabhängig und liegt vorzugsweise bei maximal pH 5.

CO₂-reiches Hydrolysegas, welches im Abluftbetrieb aus dem Perkolator abgeleitet wird, wird einem weiteren, zu diesem Perkolator zeitlich versetzt betriebenen und zu spülenden Perkolator als Spülgas zugeführt. Eine energetische Nutzung des CO₂-reichen Hydrolysegases ist nach dem erfindungsgemäßen Verfahren nicht vorgesehen.

An den Abluftbetrieb schließt sich der Gasnutzungsbetrieb an. Im Gasnutzungsbetrieb wird das Hydrolysegas aus dem Perkolator abgeleitet und einer energetischen Nutzung zugeführt. Der Gasnutzungsbetrieb wird zu dem Prozesszeitpunkt der Perkolation durchgeführt, wenn methanhaltiges Hydrolysegas im Perkolator vorliegt. Vorzugsweise wird der Gasnutzungsbetrieb so lange durchgeführt, bis die Gesamtmenge des gebildeten Hydrolysegases oder die Methankonzentration des im Perkolator befindlichen Gases jeweils zuvor festgelegte Grenzwerte unterschreiten. Das methanhaltige Hydrolysegas wird aus dem Perkolator abgeleitet und anschließend einer energetischen Nutzung zugeführt. Dafür wird es ggf. mit dem Biogas, welches in der Methanstufe des erfindungsgemäßen Biogasverfahrens gebildet wird, vereinigt.

Bei der Spülung in Betriebsstufe d. wird das im Perkolator vorliegende Hydrolysegas zunächst durch Verdrängung mit zugeleitetem Spülgas entfernt. Als Spülgas wird Abluft aus einem anderen, zeitlich versetzt betriebenen Perkolator verwendet, welcher sich zu diesem Zeitpunkt im Abluftbetrieb befindet. Dazu müssen die mindestens zwei Perkolatoren zeitlich versetzt betrieben werden, so dass sich mindestens ein Perkolator im Abluftbetrieb befindet. Unter "zeitlich versetztem Betrieb" im Sinne der Erfindung wird daher verstanden, dass mindestens zwei Perkolatoren zu unterschiedlichen Startzeitpunkten mit festen biogenen Stoffen befüllt und der Hydrolyse des Substrats ausgesetzt werden, so dass sich mindestens ein Perkolator im Abluftbetrieb befindet.

Solange die Gasatmosphäre des Perkolators energetisch nutzbare Methankonzentrationen enthält und die Methankonzentration im Perkolator mindestens einen sicherheitsrelevanten Grenzwert beträgt, wird die Gasatmosphäre der Gasnutzung (energetische Nutzung) zugeführt. Während der Spülung in Stufe d. sinkt die Methankonzentration in der Gasatmosphäre des Perkolators. Dies wird vorzugsweise so lange durchgeführt, bis ein vollständiger Austausch der im Perkolator befindlichen Gasmenge erfolgt ist und die Methankonzentration der Gasatmosphäre des Perkolators so weit verringert worden ist, dass sie einen sicherheitsrelevanten Grenzwert unterschreitet. Bevorzugt liegt der sicherheitsrelevante Grenzwert der Methankonzentration bei weniger als 1 Vol.-%, besonders bevorzugt bei 20 % der unteren Explosionsgrenze (UEG) für Methan (entspricht ca. 0,88 Vol.-%).

Bei der Spülung sinkt somit der Methananteil der Gasatmosphäre des Perkolators und der CO₂-Anteil der Gasatmosphäre steigt an. Solange der Methananteil im Gas einen zuvor definierten Grenzwert überschreitet, wird das verdrängte methanhaltige Hydrolysegas einer energetischen Nutzung zugeführt. Da im Laufe der Spülung der Methananteil im Gas stetig sinkt, ist eine energetische Nutzung des Gases bei zu geringem Methananteil nicht mehr sinnvoll. Daher wird das Gas vorzugsweise beim Unterschreiten eines zuvor definierten Grenzwerts für die Methankonzentration aus dem Perkolator abgeleitet ohne einer energetischen Nutzung zugeführt zu werden. Um diesen Prozesszeitpunkt festzustellen, erfolgt eine vorzugsweise kontinuierliche Bestimmung des Methangehalts des im Perkolator befindlichen Gases.

Zur Reduzierung des Anteils des Spülgases im Perkolator wird vor der Öffnung des Perkolators eine Spülung mit Luft vorgenommen. Dafür wird Umgebungsluft in den Perkolator eingeleitet, welche das Gasgemisch aus Spülgas und Hydrolysegas aus dem Perkolator verdrängt.

Nach der Spülung wird der Perkolator ggf. geöffnet und kann dann entleert werden und wieder mit neuen biogenen Stoffen befüllt werden. Die Öffnung erfolgt vorzugsweise dann, wenn die Methankonzentration und die CO₂-Konzentration im Perkolator jeweils unter einen zuvor definierten Schwellenwert fallen. Die Methankonzentration beträgt dabei vorzugsweise weniger als 50 % der Maximalen Arbeitsplatzkonzentration (MAK), also vorzugsweise ca. 0,5 Vol.-%.

Die Dauer der Perkolation ist abhängig von den eingesetzten biogenen Stoffen. Vorzugsweise beträgt die Dauer einer Perkolation (Vollziehen der obengenannten Verfahrensschritte a. bis f.) weniger als 30 Tage, bevorzugt 14 bis 25 Tage. Der Abluftbetrieb umfasst davon vorzugsweise die ersten 5 bis 9 Tage. Bei der Spülung eines Perkolators wird dann das in dem zu spülenden Perkolator vorliegende (methanhaltige) Hydrolysegas durch Verdrängung mit Abluft aus einem zeitlich versetzt und im Abluftbetrieb betriebenen Perkolator entfernt.

Um sicherzustellen, dass der Gasaustausch zwischen den dann verbundenen Perkolatoren bei der Spülung einseitig in Richtung des zu spülenden Perkolators läuft, wird der zeitversetzt betriebene Perkolator, welcher im Abluftbetrieb betrieben wird, vor der Spülung des zu spülenden Perkolators vorzugsweise gasseitig verschlossen, so dass ein Überdruck in diesem Perkolator (welcher im Abluftbetrieb ist) entsteht. Anschließend wird die Abluft aus dem zeitversetzt betriebenen Perkolator, vorzugsweise nach Erreichen eines Druck-Schwellenwertes, in den zu spülenden Perkolator geleitet. Der Druck-Schwellenwert ist dabei höher, als der im zu spülenden Perkolator herrschende Innendruck. Bevorzugt beträgt der Druck-Schwellenwert mindestens 5 mbar Überdruck im Vergleich zum Druck innerhalb des zu spülenden Perkolators. Dadurch wird verhindert, dass methanhaltiges Hydrolysegas aus dem zu spülenden Perkolator in den zeitversetzt im Abluftbetrieb betriebenen Perkolator gelangt und ggf. beim Ableiten als Abluft in die Atmosphäre abgegeben wird.

Die Erfindung umfasst auch eine Anlage zur zwei- oder mehrstufigen Biogasgewinnung, die zur Durchführung eines erfindungsgemäßen Verfahrens geeignet ist.

Die erfindungsgemäße Anlage zur zwei- oder mehrstufigen Biogasgewinnung umfasst mindestens zwei Perkolatoren, insbesondere Feststoffperkolatoren, welche jeweils eine Hydrolysat-Ableitung und jeweils eine Flüssigkeitszuleitung aufweisen. Die Hydrolysat-Ableitung eines Perkolators ist über mindestens einen Hydrolysat-Speicherbehälter mit mindestens einem Methanreaktor verbunden.

Die Perkolatoren in der erfindungsgemäßen Anlage sind bezüglich der Hydrolysatführung entweder parallel oder in Reihe geschaltet. Bei Parallelschaltung der Perkolatoren sind die Hydrolysat-Ableitungen der Perkolatoren vorzugsweise mit mindestens einem Hydrolysat-Speicherbehälter verbunden, in welchem das Hydrolysat der verschiedenen Perkolatoren vereint wird. Parallel geschaltete Perkolatoren weisen eine gemeinsame Flüssigkeits-Zuleitung auf. Alternativ oder zusätzlich dazu ist jedem Perkolator vorzugsweise ein separater Hydrolysat-Speicherbehälter (hierin "Hydrolysat-Vorspeicher") nachgeordnet, wobei die Hydrolysat-Vorspeicher mit dem Hydrolysat-Speicherbehälter über jeweils eine Flüssigkeitsleitung verbunden sind.

Bei Reihenschaltung der Perkolatoren sind die Hydrolysat-Ableitungen der Perkolatoren, vorzugsweise über einen Hydrolysat-Vorspeicher, mit der Flüssigkeits-Zuleitung des jeweils nächsten Perkolators, welcher zu einem früheren Zeitpunkt mit biogenen Stoffen beschickt wurde, verbunden.

Dem mindestens einen Methanreaktor ist über eine Gärflüssigkeits-Ableitung mindestens ein Gärflüssigkeits-Speicherbehälter nachgeordnet. In der erfindungsgemäßen Anlage sind Hydrolysat-Speicherbehälter und Gärflüssigkeits-Speicherbehälter mit den jeweiligen Flüssigkeitszuleitungen der Perkolatoren verbunden. Dadurch wird ermöglicht, dass ein Teil des Hydrolysats und/oder Gärflüssigkeit den Perkolatoren zur Hydrolyse als Prozesswasser zugeleitet werden können. Sind mehrere Methanreaktoren in der erfindungsgemäßen Anlage enthalten, so sind diese flüssigseitig bevorzugt mit demselben Hydrolysat-Speicherbehälter und/oder Gärflüssigkeitsspeicherbehälter verbunden.

In der erfindungsgemäßen Anlage sind die Perkolatoren gasdicht ausgestaltet sind und umfassen jeweils mindestens eine jeweils verschließbare Gas-Zuleitung und Gas-Ableitung. Jeder Perkolator ist mit einem Methansensor verbunden, welcher zur Bestimmung des Methangehalts des im Perkolator enthaltenen Gases dient. Dieser ist vorzugsweise in einer Meßeinrichtung zur Bestimmung der Gasqualität und -menge enthalten, die mit dem jeweiligen Perkolator verbunden ist. Weiter ist jeder Perkolator mit einem pH-Sensor verbunden, welcher zur Bestimmung des pH-Werts der im jeweiligen Perkolator enthaltenen Flüssigkeit dient.

Die verschließbare Gas-Zuleitung eines Perkolators in der erfindungsgemäßen Anlage ist so ausgestaltet, dass ein Umschalten auf Luftzufuhr oder auf Zufuhr von Spülgas möglich ist. Die mindestens zwei Perkolatoren sind untereinander über deren Gas-Zuleitungen verbunden, so dass bei zeitversetztem Betrieb die Einleitung von CO₂-reichem Hydrolysegas von einem Perkolator im Abluftbetrieb in einen zu spülenden Perkolator erfolgen kann. Dazu werden beide Gaszuleitungen auf Spülgaszufuhr eingestellt, so dass ein Austausch der Gasatmosphären beider Perkolatoren möglich ist.

Jeder Perkolator der erfindungsgemäßen Anlage weist die genannten Ausstattungsmerkmale auf (gasdichte Ausgestaltung mit verschließbarer Zu- und Ableitung für Gas, Methansensor). Es sind mindestens zwei, besonders bevorzugt mindestens drei Perkolatoren in einer erfindungsgemäßen Anlage enthalten.

Perkolatoren sind aus dem Stand der Technik bekannt. Sie enthalten einen Gitterrost oder einen Siebboden, auf welchem die biogenen Stoffe abgelagert werden. Weiter enthalten sie einen Zulauf für Prozesswasser bzw. Perkolat und einen Ablauf für Perkolat. Die Perkolatoren, die in einer erfindungsgemäßen Anlage eingesetzt werden, sind gasdicht abgeschlossen und enthalten eine verschließbare Zu- und Ableitung für Gas, die einen Gasaustausch mit der Umgebung nur nach gezielter Öffnung ermöglicht. Dadurch wird vermieden, dass die zu perkolierenden biogenen Stoffe dauerhaft aerober Atmosphäre ausgesetzt sind, was den Abbau zu Kohlendioxid und Wasser begünstigt. Die anaerobe Atmosphäre fördert die vermehrte Bildung der für die Methanisierung notwendigen niedermolekularen organischen Produkte.

Der Verschluss der Gasleitungen (Gas-Zuleitungen und Gas-Ableitungen), die vorzugsweise rohrförmig ausgestaltet sind, erfolgt bevorzugt durch Ventile oder Gasklappen, die an den Zu- und Ableitungen angeordnet sind.

Vorzugsweise umfasst ein Perkolator einer erfindungsgemäßen Anlage eine verschließbare Gas-Ableitung, die so ausgestaltet ist, dass ein Umschalten auf Gasableitung zu einer Anlage für die energetische Nutzung des methanhaltigen Hydrolysegases (Gasnutzungsanlage), Gasableitung in die Atmosphäre oder ein Verschluss der Gas-Ableitung möglich ist. Entsprechende Lösungen zur Gasableitung zu verschiedenen Nutzungsvarianten sind aus dem Stand der Technik bekannt. Vorzugsweise enthält die Gasableitung dafür speziell ausgestaltete Ventile oder Gasklappen, die das Umschalten ermöglichen. Besonders bevorzugt ist dafür an der Gas-Ableitung ein verschließbares Zweiwegeventil angeordnet.

Bevorzugt ist die Gasnutzungsanlage ein Blockheizkraftwerk, eine Anlage zur Herstellung von Biomethan (CO₂-Wäsche) oder der Methanreaktor der erfindungsgemäßen Anlage. Vorzugsweise ist vor der Gasnutzungsanlage ein Gasspeicher angeordnet.

Die erfindungsgemäße Anlage ist so ausgestaltet, dass die Flüssigkeitszuleitung eines Perkolators zur Zuleitung von Hydrolysat und Gärflüssigkeit in den Perkolator geeignet ist. Dafür ist die Flüssigkeitszuleitung mit einem Hydrolysat-Speicherbehälter und mit einem Gärflüssigkeits-Speicherbehälter verbunden. Diese Ausbildung der erfindungsgemäßen Anlage ermöglicht den Kreislaufbetrieb der Flüssigkeiten (Hydrolysat, Gärflüssigkeit) im erfindungsgemäßen Verfahren.

Erfindungsgemäße Anlagen enthalten mindestens zwei, bevorzugt mindestens drei, gasdicht ausgestaltete Perkolatoren, die entweder parallel oder in Reihe geschaltet sind und die untereinander über deren Gas-Zuleitungen verbunden sind, so dass bei zeitversetztem Betrieb die Einleitung von CO₂-reichem Hydrolysegas von einem Perkolator im Abluftbetrieb in einen zu spülenden Perkolator erfolgen kann. In parallel geschalteten Perkolatoren wird die Hydrolysatflüssigkeit über die Hydrolysat-Ableitung in einem Hydrolysat-Speicherbehälter gesammelt und vereint (flüssigseitige Parallelschaltung). Im Gegensatz zu in Reihe geschalteten Perkolatoren, bei denen das Hydrolysat von einem Perkolator in einen zeitlich versetzt betriebenen, zu einem früheren Zeitpunkt mit biogenen Stoffen beschickten Perkolator zugeleitet wird, werden die Hydrolysat-Flüssigkeiten der einzelnen Perkolatoren in Parallelschaltung vereinigt.

Die Perkolatoren sind untereinander mit einer verschließbaren Gasleitung verbunden, die die jeweilige Gas-Zuleitung des Perkolators darstellt. Die Gas-Zuleitungen sind verschließbar und so ausgestaltet, dass ein Umschalten von Luftzufuhr, Zufuhr von Gas aus einem anderen Perkolator oder ein Verschluss der Gas-Zuleitung möglich ist. Durch diesen Aufbau ist ein gezielter Gasaustausch zwischen den Perkolatoren möglich. Als Verschluss dienen vorzugsweise Ventile oder Gasklappen.

Die Perkolatoren werden zeitlich versetzt betrieben, d.h. dass die Beschickung mit jeweils frischen biogenen Stoffen in bevorzugt gleichen Abständen zeitversetzt erfolgt. Das in den Perkolatoren gebildete flüssige Hydrolysat wird aus dem jeweiligen Perkolator über eine Hydrolysat-Ableitung in einen Hydrolysat-Speicherbehälter geleitet. Von dort aus wird ein Teil der Flüssigkeit in den Methanreaktor der erfindungsgemäßen Anlage geführt.

In weiter bevorzugten erfindungsgemäßen Anlagen ist an den Perkolatoren jeweils ein Drucksensor angeordnet, der zur Bestimmung des Drucks innerhalb des Perkolators dient.

Die erfindungsgemäße Anlage wird wie folgt betrieben:
Mindestens zwei Perkolatoren werden zeitlich versetzt mit festen biogenen Stoffen beschickt und geschlossen. Über den Flüssigkeits-Zulauf werden die festen biogenen Stoffe (Substrat) in einem Perkolator von der im Kreislauf gefahrenen Prozessflüssigkeit (Hydrolysat und Gärflüssigkeit) berieselt und durchströmt. Durch das Eintragen der Flüssigkeit findet eine biochemische Umsetzung der abbaubaren Bestandteile des Substrats zu Alkoholen, Zuckern und niederen Fettsäuren statt, welche so in eine wasserlösliche Form überführt werden und mit dem flüssigen Hydrolysat aus dem Perkolator über die Hydrolysat-Ableitung ausgetragen werden. Durch den Gitterrost oder Siebboden des Perkolators wird das feste Substrat dabei zurückgehalten.

Der Kreislaufbetrieb wird dadurch realisiert, dass Flüssigkeit aus dem Hydrolysat-Speicherbehälter und/oder dem Gärflüssigkeits-Speicherbehälter über die Flüssigkeits-Zuleitung in den Perkolator eingetragen wird. Anschließend wird das Hydrolysat in einen Hydrolysat-Speicherbehälter überführt, und dann kontinuierlich dem Methanreaktor zugeführt, wo eine Vergärung zu methan- und kohlendioxidhaltigem Biogas erfolgt.

Durch den gasdichten Abschluss der Perkolatoren wird das Eindringen von Luftsauerstoff in den jeweiligen Perkolator sowie das unkontrollierte Entweichen von Hydrolysegas verhindert.

Während der Perkolation wird Hydrolysegas gebildet, dessen chemische Zusammensetzung über den Perkolationszeitraum variiert. Nach der Beschickung eines Perkolators mit biogenen Stoffen wird zu Beginn der Perkolation ein CO₂-reiches Hydrolysegas gebildet. Bei fortschreitender Perkolation sinkt der CO₂-Gehalt des Hydrolysegases. Gleichzeitig steigt der Methangehalt des Hydrolysegases. Während des Prozesses werden zunächst u.a. organische Säuren als Umwandlungsprodukte der biogenen Stoffe hergestellt, so dass der pH-Wert des Perkolats im sauren Milieu liegt. Mit fortschreitender Perkolation steigt der pH-Wert in den leicht sauren bis neutralen Bereich.

In einem Perkolator werden bevorzugt folgende Betriebsstufen durchgeführt:
Abluftbetrieb: Das zu Beginn der Perkolation in einem Perkolator gebildete, CO₂-reiche Hydrolysegas kann nicht energetisch genutzt werden und wird über die Gasableitung aus dem Perkolator abgeleitet. Zu diesem Zeitpunkt ist der pH-Wert des Hydrolysats im stark sauren Milieu. An der Gas-Ableitung wird ein Ventil geöffnet, so dass das Hydrolysegas als Abluft aus dem Perkolator abgeleitet wird.

Gasnutzungsbetrieb: Im weiteren Verlauf der Perkolation entsteht zunehmend Methan, so dass der Methananteil am Hydrolysegas steigt, während der CO₂-Anteil abnimmt. Übersteigt der mit dem Methansensor der Meßeinrichtung bestimmte Methangehalt am Hydrolysegas einen definierten Grenzwert, wird die Gas-Ableitung zu umgeschaltet, dass die Gas-Ableitung mit einer Gasnutzungsanlage verbunden ist. Vorzugsweise wird das Hydrolysegas vorher in einem Speicher gesammelt und in einer Gasaufbereitungsanlage aufbereitet. Zu diesem Zeitpunkt ist der pH-Wert des Hydrolysats im neutralen bis schwach sauren Milieu.

Spülung mit Spülgas: Zum Ende der Perkolation werden nur noch geringe Mengen organischer Abbauprodukte der biogenen Stoffe im Hydrolysat über den Hydrolysat-Ablauf ausgetragen. Der pH-Wert am Hydrolysat-Ablauf nähert sich dem pH-Wert des Flüssigkeits-Zulaufs an. Die Bestimmung des pH-Werts der Flüssigkeit erfolgt über pH-Sensoren in der Flüssigkeits-Zuleitung und Hydrolysat-Ableitung. Der Methangehalt des Hydrolysegases ist jedoch weiterhin sehr hoch. Zum Austreiben des restlichen methanhaltigen Hydrolysegases aus einem Perkolator wird als Spülgas Abluft aus einem (zu dem zu spülenden Perkolator) zeitlich versetzt betriebenen weiteren Perkolator über die Gas-Zuleitung bei geöffnetem Ventil eingetragen. Über ein geöffnetes Ventil in der Gas-Ableitung wird die Gasmischung aus Hydrolysegas und Spülgas aus dem Perkolator zur Gasnutzungsanlage geleitet.

Erreicht die Methankonzentration des Gasgemischs aus Hydrolysegas und zugeströmten Spülgas innerhalb des Perkolators einen zuvor definierten unteren Grenzwert, wird die Spülung mit Luft eingeleitet. Die Methankonzentration des Gasgemischs in Perkolator wird dabei über die Meßeinrichtung ermittelt.

Spülung mit Luft: Über die Gas-Zuleitung wird Umgebungsluft eingetragen. Über die Meßeinrichtung wird die Gaszusammensetzung im Perkolator bestimmt. Wenn die durch die Meßeinrichtung ermittelte Methankonzentration- und die Konzentration des CO₂ einen zuvor definierten jeweiligen Minimalwert erreicht hat, wird signalisiert, dass der Perkolator geöffnet, entleert und neu beschickt werden kann. Mit der Neubeschickung beginnt der Zyklus aus Abluftbetrieb, Gasnutzungsbetrieb, Spülung mit Spülgas, Spülung mit Luft und Öffnung von neuem.

Das erfindungsgemäße Verfahren bietet die Möglichkeit, die energetisch nicht nutzbare Fraktion des Hydrolysegases (das CO₂-reiche Hydrolysegas) einer Nutzung innerhalb des Prozesses zuzuführen, indem es für die Spülung von zeitlich versetzt betriebenen Perkolatoren verwendet wird.

Zusätzlich dazu kann mit dem erfindungsgemäßen Verfahren die methanhaltige Fraktion des Hydrolysegases (methanhaltiges Hydrolysegas) energetisch genutzt werden. Durch die Überwachung des Methangehalts im Hydrolysegas und die unterschiedliche Nutzung der Hydrolysegase in Abhängigkeit der darin enthaltenen Gase (Methan, CO₂) wird verhindert, dass ein bedeutender Teil des energetisch nicht nutzbaren CO₂ in den Biogasstrom gelangt. Es wird damit außerdem verhindert, dass energetisch nutzbares Methan bei der Biogasgewinnung ungenutzt in die Atmosphäre entlassen wird. Dadurch wird einerseits eine verbesserte ökonomische Nutzung der umgewandelten biogenen Stoffe sichergestellt und weiter wird verhindert, dass methanhaltiges Gas bei der Öffnung gasdichter Perkolatoren unkontrolliert aus dem Perkolator entweicht und damit ein Sicherheitsrisiko darstellt oder klimaschädliche Wirkung entfaltet. Durch die Abtrennung eines Teils der CO₂-reichen Fraktion des Hydrolysegases wird andererseits die energetische Qualität des Rohbiogases der Gesamtanlage erhöht, was Vorteile bei der weiteren Gasverwertung bietet.

Durch die Nutzung des entstehenden Hydrolysegases in entsprechenden gasdicht ausgestalteten Perkolatoren wird vorteilhaft ein besserer Umsatz der eingesetzten biogenen Stoffe zu energetisch nutzbarem Biogas erzielt und deren energetisch unvorteilhafte Umwandlung zu Kohlendioxid und Wasser im Perkolator verringert.

So wird mit einem erfindungsgemäßen Verfahren bzw. einer entsprechenden erfindungsgemäßen Anlage im Vergleich zu konventionellen ein- und zweistufigen Biogasverfahren eine höhere Methanausbeute aus den eingesetzten biogenen Stoffen erzielt und eine sichere und umweltschonende Betriebsweise der Biogasanlage ermöglicht.

Wird das Verfahren in einer Anlage mit mehreren Methanreaktoren durchgeführt, so kann das Verfahren und die Leistung der Anlage zudem variabler gesteuert werden. In Abhängigkeit vom Bedarf, beispielsweise in Abhängigkeit von der Menge der eingesetzten biogenen Stoffe, kann die Leistung durch das Stilllegen oder Zuschalten von Methanreaktoren reguliert werden. Innerhalb der Anlage ist ein gleichmäßiger Betrieb möglich, da auch bei Stillstand (beispielsweise bei der Wartung) eines Methanreaktors dennoch der Betrieb aufrecht erhalten werden kann.

Anhand folgender Figuren und Ausführungsbeispiele soll die Erfindung näher erläutert werden, ohne die Erfindung auf diese zu beschränken.

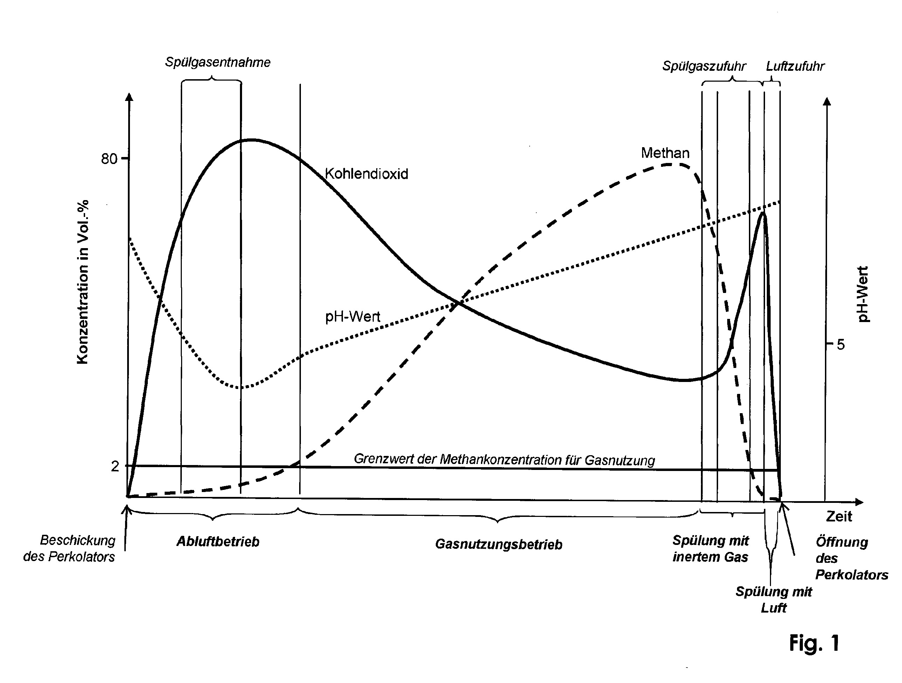

- Fig. 1: Schematische Darstellung der CO₂- und Methan-Konzentration, sowie pH-Wert-Entwicklung in einem Perkolator einer erfindungsgemäßen Anlage
- Fig. 2: Schema vier parallel geschalteter gasdichter Perkolatoren einer Anlage zur zweistufigen Biogasgewinnung mit einem erfindungsgemäßen Verfahren
- Fig. 3: Schema vier in Reihe geschalteter gasdichter Perkolatoren einer Anlage zur zweistufigen Biogasgewinnung mit einem erfindungsgemäßen Verfahren
- Fig. 4: Schema vier parallel geschalteter gasdichter Perkolatoren einer Anlage zur zweistufigen Biogasgewinnung mit einem erfindungsgemäßen Verfahren mit zwei Methanreaktoren
- Fig. 5: Schema vier in Reihe geschalteter gasdichter Perkolatoren einer Anlage zur zweistufigen Biogasgewinnung mit einem erfindungsgemäßen Verfahren mit zwei Methanreaktoren

### Beispiel 1: Erfindungsgemäße Anlage zur zweistufigen Biogasgewinnung mit vier Perkolatoren in Parallelschaltung

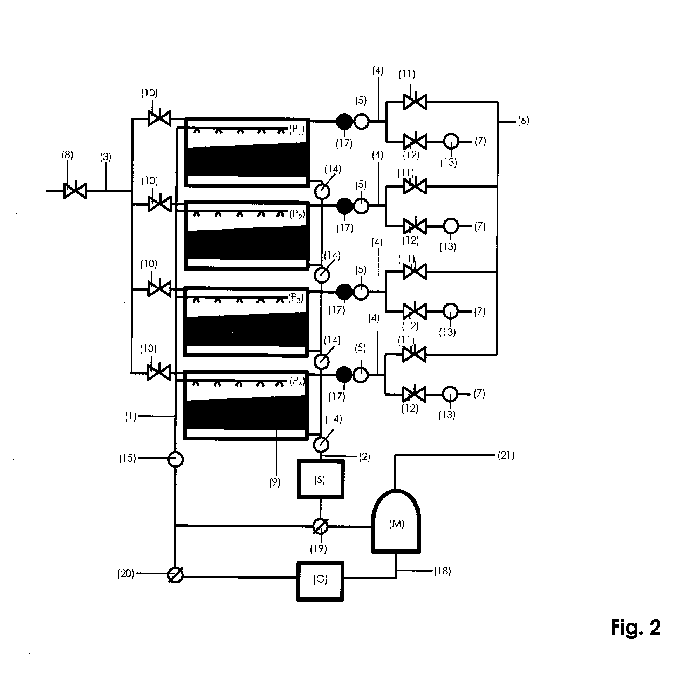

Die erfindungsgemäße Anlage umfasst vier Hydrolysereaktoren (P₁-P₄), in diesem Fall Feststoffperkolatoren, wie in Fig. 2 dargestellt und einen Methanreaktor. Die Perkolatoren (P₁-P₄) sind gasdicht abgeschlossen und enthalten jeweils eine Gas-Zuleitung (3) und jeweils eine Gas-Ableitung (4), die im oberen Teil des jeweiligen Perkolators, vorzugsweise auf entgegengesetzten Seiten, angebracht sind.

Die Perkolatoren (P₁-P₄) sind jeweils mit einem Gitterrost oder Siebboden (9) ausgestattet, auf dem die zu perkolierenden festen biogenen Stoffe abgelagert werden. Weiter enthält jeder Perkolator (P₁-P₄) am oberen Ende eine Flüssigkeits-Zuleitung (1), durch welchen die Flüssigkeit eingetragen wird, mit der die festen biogenen Stoffe berieselt werden. Unterhalb des Gitterrosts oder Siebbodens (9) eines Perkolators (P₁-P₄) ist jeweils der Hydrolysat-Ableitung (2) angebracht. Sowohl Flüssigkeits-Zuleitung (1) als auch die Hydrolysat-Ableitung (2) sind mit jeweils einem pH-Sensor (14, 15) verbunden, der zur Bestimmung des pH-Werts der sich in der Leitung befindenden Flüssigkeit dient.

Die Hydrolysat-Abläufe (2) der Perkolatoren (P₁-P₄) sind miteinander verbunden und sind über einen Hydrolysat-Speicherbehälter (S) an den Methanreaktor (M) angeschlossen. Zwischen Hydrolysat-Speicherbehälter (S) und Methanreaktor (M) ist ein Ventil (19) angebracht, dass je nach dessen Einstellung ermöglicht, dass Flüssigkeit in den Methanreaktor (M) oder in den Perkolator (P) (über die Flüssigkeitszuleitung (1)) gelangt.

Der Methanreaktor (M) umfasst eine Biogasableitung (21). Dem Methanreaktor (M) ist über die Gärflüssigkeits-Ableitung (18) ein Gärflüssigkeits-Speicherbehälter (G) nachgeordnet.

Der Flüssigkeits-Zulauf (1) der Perkolatoren (P₁-P₄) ist mit dem Gärflüssigkeits-Speicherbehälter (G) und dem Hydrolysat-Speicherbehälter (S) verbunden. Im Flüssigkeits-Zulauf (1) ist ein pH-Sensor (15) zur Bestimmung der pH-Werts der zugeleiteten Flüssigkeit angeordnet. Im Flüssigkeits-Zulauf (1) ist weiterhin ein Ventil (20) angeordnet.

Die Gas-Zuleitung (3) ist mit einem Ventil (8) ausgestattet und kann gezielt geöffnet bzw. verschlossen werden, so dass Umgebungsluft in die Leitung eindringen kann. Die Gas-Zuleitungen (3) der einzelnen Perkolatoren (P₁-P₄) sind untereinander verbunden und weisen jeweils vor der Mündung in jeden Perkolator (P₁-P₄) eine Gasklappe (10) auf, die jeweils separat geöffnet werden können. Durch Öffnung der Gasklappen (10) zweier Perkolatoren ist ein gegenseitiger Gasaustausch der beiden Perkolatoren möglich.

Die Gas-Ableitung (4) der einzelnen Perkolatoren (P₁-P₄) ist jeweils mit einer Meßeinrichtung (5) verbunden, die zur Bestimmung der Gasqualität und Gasmenge im jeweiligen Perkolator (P₁-P₄) dient, und den Methangehalt des im Perkolator (P₁-P₄) befindlichen Gases bestimmt. Die Gas-Ableitung (4) verzweigt sich und ist jeweils mit zwei Ventilelementen (11, 12) ausgestattet, welche mit unterschiedlichen Funktionseinheiten der Anlage verbunden sind.

Die Ventile (11) sind mit einer Leitung zur Anlage zur energetischen Nutzung von methanhaltigem Hydrolysegas (6) verbunden, wobei ggf. ein Lagerungsbehälter für das methanhaltige Hydrolysegas und/oder eine Gasaufbereitungsanlage zwischengeschaltet ist.

Die Ventile (12) sind jeweils über ein Abluftgebläse (13) mit einer Abluftanlage (7) verbunden. So wird eine Absaugung der Abluft ermöglicht.

Jeder Perkolator (P₁-P₄) ist mit einem Drucksensor (17) ausgestattet.

Die Anlage wird wie folgt betrieben: Die Perkolatoren (P₁-P₄) werden zeitversetzt mit festen biogenen Stoffen beschickt. Die Perkolatoren (P₁-P₄) werden jeweils geschlossen. Über die Flüssigkeits-Zuleitung (1) werden die festen biogenen Stoffe (Substrat) im Perkolator (P₁-P₄) von der im Kreislauf gefahrenen Perkolationsflüssigkeit (Perkolat und Gärflüssigkeit) berieselt und durchströmt. Durch das Eintragen der Flüssigkeit findet eine biochemische Umsetzung der abbaubaren Bestandteile des Substrats zu Alkoholen, Zuckern und niederen Fettsäuren statt, welche so in eine wasserlösliche Form überführt werden und mit dem flüssigen Perkolat aus dem Perkolator über die Hydrolysat-Ableitung (2) ausgetragen werden. Durch den Gitterrost oder Siebboden (9) des Perkolators (P₁-P₄) wird das feste Substrat dabei zurückgehalten.

Der Kreislaufbetrieb wird dadurch realisiert, dass Flüssigkeit aus dem Hydrolysat-Speicherbehälter (S) und/oder dem Gärflüssigkeits-Speicherbehälter (G) über die Flüssigkeits-Zuleitung (1) in die Perkolatoren (P₁-P₄) eingetragen wird.

Anschließend wird das Hydrolysat in einen Hydrolysat-Speicherbehälter (S) überführt. Von dort wird es entweder erneut zur Perkolation verwendet (durch Eintragen in die Perkolatoren (P₁-P₄) über die Flüssigkeits-Zuleitung (1)) oder im Teilstrom kontinuierlich dem Methanreaktor (M) zugeführt, wo eine Vergärung zu methan- und kohlendioxidhaltigem Biogas erfolgt.

Zum Ende der Perkolation sinkt die Menge der in der Hydrolysatflüssigkeit gelösten organischen Bestandteile der biogenen Stoffe. Daher werden mehrere Perkolatoren (P₁-P₄) zeitversetzt beschickt und die über die jeweiligen Hydrolysat-Ableitungen (2) ausfließenden Hydrolysate in dem Hydrolysat-Speicherbehälter (S) vereinigt. Dadurch kann sichergestellt werden, dass eine gleichmäßige Zufuhr von organischen Abbauprodukten der biogenen Stoffe zum Methanreaktor erfolgt.

Durch die gasdichte Ausführung der Perkolatoren (P₁-P₄) wird das Eindringen von Luftsauerstoff in die Perkolatoren (P₁-P₄) sowie das unkontrollierte Entweichen von Hydrolysegas verhindert.

Das Hydrolysegas wird über die Gas-Ableitung (4) entnommen. Über die Meßeinrichtung (5) wird der Methan- und Kohlendioxidgehalt des Hydrolysegases kontrolliert.

Abluftbetrieb: Zu Beginn der Perkolation ist das Hydrolysegas CO₂-reich. Zu diesem Zeitpunkt ist der pH-Wert im Hydrolysat im stark sauren Milieu. Das Ventil (12) wird geöffnet und das Abluftgebläse (13) wird aktiviert. Das CO₂-reiche Hydrolysegas wird aus dem Perkolator (P₁-P₄) abgeleitet.

Gasnutzungsbetrieb: Im weiteren Verlauf der Perkolation entsteht zunehmend Methan, so dass der Methananteil am Hydrolysegas steigt, während der CO₂-Anteil abnimmt. Übersteigt der mit der Meßeinrichtung (5) bestimmte Methangehalt am Hydrolysegas einen definierten Grenzwert, wird das Ventil (12) verschlossen und das Ventil (11) geöffnet. Das methanreiche Hydrolysegas wird in eine Anlage zur energetischen Nutzung (6) geleitet. Vorzugsweise wird es vorher in einem Speicher gesammelt und in einer Gasaufbereitungsanlage aufbereitet. Zu diesem Zeitpunkt ist der pH-Wert des Hydrolysats im neutralen bis schwach sauren Milieu. Die Bestimmung des pH-Werts erfolgt über den jeweiligen pH-Sensor (14) der Perkolatoren (P₁-P₄).

Spülung mit inertem Gas: Zum Ende der Perkolation werden nur noch geringe Mengen organischer Abbauprodukte der biogenen Stoffe im Hydrolysat über den Ablauf (2) ausgetragen. Der pH-Wert an der Hydrolysat-Ableitung (2) nähert sich dem pH-Wert des Flüssigkeits-Zulaufs (1) an. Die Bestimmung des pH-Werts der Flüssigkeit erfolgt über pH-Sensoren (14, 15).) Es entsteht jedoch weiterhin Hydrolysegas, zwar in geringeren Mengen, jedoch mit einem signifikanten Methananteil (methanhaltiges Hydrolysegas). Zum Austreiben (Verdrängen) des restlichen methanhaltigen Hydrolysegases aus einem Perkolator (P₁) wird die CO₂-reiche Abluft (CO₂-reiches Hydrolysegas) eines zeitversetzt betriebenen und sich gerade im Abluftbetrieb befindlichen Perkolators (P₂) eingesetzt.

Dafür wird zunächst die Gasklappe (10) des Perkolators (P₁) manuell oder mit Hilfe eines Prozessleitsystems geschlossen. Das Ventil (12) mit Verbindung zur Gasnutzungsanlage (6) des Perkolators (P₁) ist weiterhin geöffnet.

An Perkolator (P₂) wird das Ventil (11) geschlossen (das Ventil (12) ist ebenfalls geschlossen), so dass der Druck innerhalb von Perkolator (P₂) durch die kontinuierlich erfolgende Gasproduktion zu steigen beginnt. Der Drucksensor (17) ermittelt vorzugsweise kontinuierlich den Gassystemdruck innerhalb des Perkolators (P₂). Übersteigt der Gassystemdruck einen zuvor definierten Druck-Schwellenwert, öffnen die Gasklappen (10) der Perkolatoren (P₁ und P₂). Die Perkolatoren (P₁ und P₂) sind somit kopfseitig miteinander verbunden.

Aufgrund des Überdrucks innerhalb des Perkolators (P₂) findet ein gerichteter Gasaustausch aus Perkolator (P₂) in Perkolator (P₁) statt. Über das geöffnete Ventil (12) wird die Gasmischung aus Perkolator (P₁) zur Gasnutzungsanlage (6) geleitet.

Erreicht die Methankonzentration des Gasgemischs aus Hydrolysegas und zugeströmten CO₂-reichem Gas innerhalb des Perkolators (P₁) einen zuvor definierten unteren Grenzwert, wird das Ventil (12) geschlossen. Die Methankonzentration des Gasgemischs in Perkolator (P₁) wird dabei über die Meßeinrichtung (5) ermittelt.

Für den Fall, dass die Methankonzentration den unteren Grenzwert nicht erreicht, kann das Ventil (12) auch manuell geschlossen werden.

Spülung mit Luft: Die Zuluftklappen (10) der Perkolatoren (P₃, P₄) werden geschlossen, sollten sich diese nicht ebenfalls im gleichen Prozessstadium befinden. Das Ventil (11) des Perkolators (P₁) und das Ventil (8) an der Gaszufuhrleitung (3) wird geöffnet, jedoch wird nunmehr über die Gaszufuhrleitung (3) Umgebungsluft eingetragen.

Das Abluftgebläse (13) wird eingeschaltet und dient zum Absaugen des im Perkolator (P₁) enthaltenen Gases. Über die Meßeinrichtung (5) wird die Gaszusammensetzung im Perkolator (P₁) bestimmt. Wenn die durch die Meßeinrichtung (5) ermittelte Methankonzentration- und die Konzentration des inerten Gases einen zuvor definierten jeweiligen Minimalwert (vorzugsweise weniger als 1 Vol.-% für Methan, vorzugsweise weniger als 1,5 Vol.-% für CO₂) erreicht hat, wird signalisiert, dass der Perkolator (P₁) geöffnet, entleert und neu beschickt werden kann.

Mit der Neubeschickung beginnt der Zyklus aus Abluftbetrieb, Gasnutzungsbetrieb, Spülung und Öffnung von neuem.

### Beispiel 2: Erfindungsgemäße Anlage zur zweistufigen Biogasgewinnung mit vier Perkolatoren in Reihenschaltung

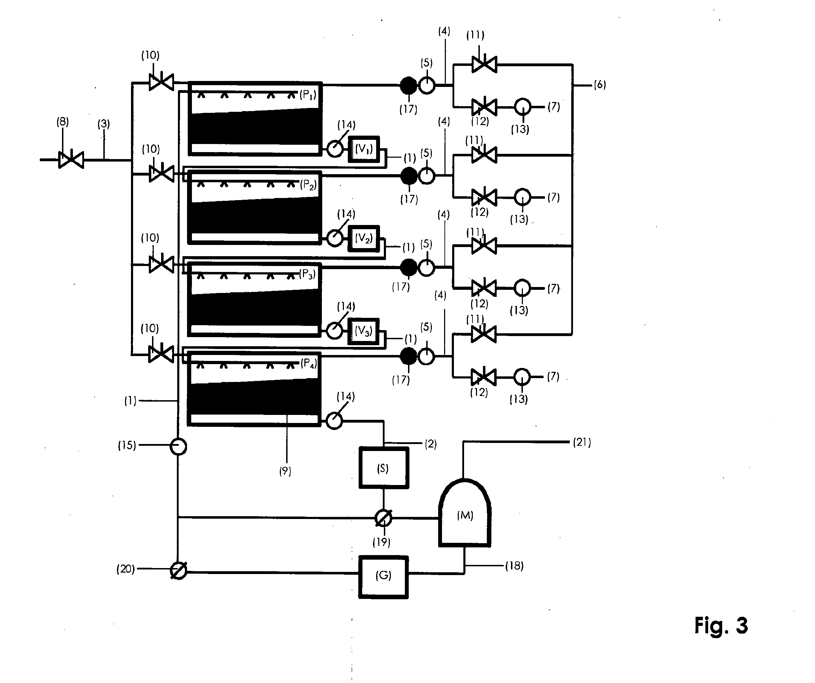

Die erfindungsgemäße Anlage umfasst vier Hydrolysereaktoren (P₁-P₄), in diesem Fall Feststoffperkolatoren, wie in Fig. 3 dargestellt und einen Methanreaktor. Die Perkolatoren (P₁-P₄) sind gasdicht abgeschlossen und enthalten jeweils eine Gas-Zuleitung (3) und jeweils eine Gas-Ableitung (4), die im oberen Teil des jeweiligen Perkolators, vorzugsweise auf entgegengesetzten Seiten, angebracht sind.

Die Perkolatoren (P₁-P₄) sind jeweils mit einem Gitterrost oder Siebboden (9) ausgestattet, auf dem die zu perkolierenden festen biogenen Stoffe abgelagert werden. Weiter enthält jeder Perkolator (P₁-P₄) am oberen Ende eine Flüssigkeits-Zuleitung (1), durch welchen die Flüssigkeit eingetragen wird, mit der die festen biogenen Stoffe berieselt werden. Unterhalb des Gitterrosts oder Siebbodens (9) eines Perkolators (P₁-P₄) ist jeweils die Hydrolysat-Ableitung (2) angebracht. Die Hydrolysat-Ableitungen (2) sind mit jeweils einem pH-Sensor (14) verbunden, der zur Bestimmung des pH-Werts der sich in der Leitung befindenden Flüssigkeit dient.

Die Hydrolysat-Abläufe (2) der Perkolatoren (P₁-P₃) sind mit jeweils einem Hydrolysat-Vorspeicher (V₁-V₃) verbunden. Die Flüssigkeits-Zuleitung (1) des Perkolators (P₂) ist mit dem Hydrolysat-Vorspeicher (V₁) verbunden. Analog dazu ist die Flüssigkeits-Zuleitung (1) des Perkolators (P₃) mit dem Hydrolysat-Vorspeicher (V₂) und die Flüssigkeits-Zuleitung (1) des Perkolators (P₄) mit dem Hydrolysat-Vorspeicher (V₃) verbunden.

Die Hydrolysat-Ableitung (2) des Perkolators (P₄) ist über einen Hydrolysat-Speicherbehälter (S) an den Methanreaktor (M) angeschlossen. Zwischen Hydrolysat-Speicherbehälter (S) und Methanreaktor (M) ist ein Ventil (19) angebracht, dass je nach dessen Einstellung ermöglicht, dass Flüssigkeit in den Methanreaktor (M) oder in den Perkolator (P) (über die Flüssigkeitszuleitung (1)) gelangt.

Der Methanreaktor (M) umfasst eine Biogasableitung (21). Dem Methanreaktor (M) ist über die Gärflüssigkeits-Ableitung (18) ein Gärflüssigkeits-Speicherbehälter (G) nachgeordnet.

Der Flüssigkeits-Zulauf (1) des Perkolators (P₁) ist mit dem Gärflüssigkeits-Speicherbehälter (G) und dem Hydrolysat-Speicherbehälter (S) verbunden. Im Flüssigkeits-Zulauf (1) ist ein pH-Sensor (15) zur Bestimmung der pH-Werts der zugeleiteten Flüssigkeit angeordnet. Im Flüssigkeits-Zulauf (1) ist weiterhin ein Ventil (20) angeordnet.

Die Gas-Zuleitung (3) ist mit einem Ventil (8) ausgestattet und kann gezielt geöffnet bzw. verschlossen werden, so dass Umgebungsluft in die Leitung eindringen kann. Die Gas-Zuleitungen (3) der einzelnen Perkolatoren (P₁-P₄) sind untereinander verbunden und weisen jeweils vor der Mündung in jeden Perkolator (P₁-P₄) eine Gasklappe (10) auf, die jeweils separat geöffnet werden können. Durch Öffnung der Gasklappen (10) zweier Perkolatoren ist ein gegenseitiger Gasaustausch der beiden Perkolatoren möglich.

Die Gas-Ableitung (4) der einzelnen Perkolatoren (P₁-P₄) ist jeweils mit einer Meßeinrichtung (5) verbunden, die zur Bestimmung der Gasqualität und Gasmenge im jeweiligen Perkolator (P₁-P₄) dient, und den Methangehalt des im Perkolator (P₁-P₄) befindlichen Gases bestimmt. Die Gas-Ableitung (4) verzweigt sich und ist jeweils mit zwei Ventilelementen (11, 12) ausgestattet, welche mit unterschiedlichen Funktionseinheiten der Anlage verbunden sind.

Die Ventile (11) sind mit einer Leitung zur Anlage zur energetischen Nutzung von methanhaltigem Hydrolysegas (6) verbunden, wobei ggf. ein Lagerungsbehälter für das methanhaltige Hydrolysegas und/oder eine Gasaufbereitungsanlage zwischengeschaltet ist.

Die Ventile (12) sind jeweils über ein Abluftgebläse (13) mit einer Abluftanlage (7) verbunden, So wird eine Absaugung der Abluft ermöglicht.

Jeder Perkolator (P₁-P₄) ist mit einem Drucksensor (17) ausgestattet.

Die Anlage wird wie folgt betrieben: Die Perkolatoren (P₁-P₄) werden zeitversetzt mit festen biogenen Stoffen beschickt. Dabei wird Perkolator (P₄) zuerst beschickt, es folgen die Perkolatoren (P₃-P₁) in dieser Reihenfolge. Die Perkolatoren (P₁-P₄) werden jeweils nach der Beschickung geschlossen.

Über die Flüssigkeits-Zuleitung (1) werden die festen biogenen Stoffe (Substrat) im Perkolator (P₁-P₄) von der im Kreislauf gefahrenen Perkolationsflüssigkeit (Perkolat und Gärflüssigkeit) berieselt und durchströmt. Durch das Eintragen der Flüssigkeit findet eine biochemische Umsetzung der abbaubaren Bestandteile des Substrats zu Alkoholen, Zuckern und niederen Fettsäuren statt, welche so in eine wasserlösliche Form überführt werden und mit dem flüssigen Perkolat aus dem Perkolator über die Hydrolysat-Ableitung (2) ausgetragen werden. Durch den Gitterrost oder Siebboden (9) des Perkolators (P₁-P₄) wird das feste Substrat dabei zurückgehalten.

Der Kreislaufbetrieb wird dadurch realisiert, dass Flüssigkeit aus dem Hydrolysat-Speicherbehälter (S) und/oder dem Gärflüssigkeits-Speicherbehälter (G) über die Flüssigkeits-Zuleitung (1) in den Perkolator (P₁) eingetragen wird. Durch die Reihenschaltung der Perkolatoren wird jeweils das Hydrolysat, welches aus einem Perkolator abgeführt wird, einem Perkolator, welcher zu einem früheren Zeitpunkt mit biogenen Stoffen beschickt wurde, zugeleitet wird. Dafür wird Hydrolysat aus Perkolator (P₁) über den Hydrolysat-Vorspeicher (V₁) in den Perkolator (P₂) überführt. Analog geschieht dies für die Zuleitung von Flüssigkeit zu den Perkolatoren (P₃) und (P₄).

Das Hydrolysat aus Perkolator (P₄) wird in einen Hydrolysat-Speicherbehälter (S) überführt. Von dort wird es entweder erneut zur Perkolation verwendet (durch Eintragen in den Perkolatoren (P₁) über die Flüssigkeits-Zuleitung (1) von (P₁)) oder im Teilstrom kontinuierlich dem Methanreaktor (M) zugeführt, wo eine Vergärung zu methan- und kohlendioxidhaltigem Biogas erfolgt.

Zum Ende der Perkolation sinkt die Menge der in der Hydrolysatflüssigkeit gelösten organischen Bestandteile der biogenen Stoffe. Daher werden mehrere Perkolatoren (P₁-P₄) zeitversetzt beschickt. Dadurch kann sichergestellt werden, dass eine gleichmäßige Zufuhr von organischen Abbauprodukten der biogenen Stoffe zum Methanreaktor erfolgt.

Durch die gasdichte Ausführung der Perkolatoren (P₁-P₄) wird das Eindringen von Luftsauerstoff in die Perkolatoren (P₁-P₄) sowie das unkontrollierte Entweichen von Hydrolysegas verhindert.

Das Hydrolysegas wird den Perkolatoren (P₁-P₄) über die Gas-Ableitung (4) entnommen. Über die Meßeinrichtung (5) wird der Methan- und Kohlendioxidgehalt des Hydrolysegases kontrolliert.

Abluftbetrieb: Zu Beginn der Perkolation ist das Hydrolysegas CO₂-reich. Zu diesem Zeitpunkt ist der pH-Wert im Hydrolysat im stark sauren Milieu. Das Ventil (12) wird geöffnet und das Abluftgebläse (13) wird aktiviert. Das CO₂-reiche Hydrolysegas wird aus dem Perkolator (P₁-P₄) abgeleitet.

Gasnutzungsbetrieb: Im weiteren Verlauf der Perkolation entsteht zunehmend Methan, so dass der Methananteil am Hydrolysegas steigt, während der CO₂-Anteil abnimmt. Übersteigt der mit der Meßeinrichtung (5) bestimmte Methangehalt am Hydrolysegas einen definierten Grenzwert, wird das Ventil (12) verschlossen und das Ventil (11) geöffnet. Das methanreiche Hydrolysegas wird in eine Anlage zur energetischen Nutzung (6) geleitet. Vorzugsweise wird es vorher in einem Speicher gesammelt und in einer Gasaufbereitungsanlage aufbereitet. Zu diesem Zeitpunkt ist der pH-Wert des Hydrolysats im neutralen bis schwach sauren Milieu. Die Bestimmung des pH-Werts erfolgt über den jeweiligen pH-Sensor (14) der Perkolatoren (P₁-P₄).

Spülung mit inertem Gas: Zum Ende der Perkolation werden nur noch geringe Mengen organischer Abbauprodukte der biogenen Stoffe im Hydrolysat über den Ablauf (2) ausgetragen. Der pH-Wert an der Hydrolysat-Ableitung (2) nähert sich dem pH-Wert des Flüssigkeits-Zulaufs (1) an. Die Bestimmung des pH-Werts der Flüssigkeit erfolgt über pH-Sensoren (14, 15). Es entsteht jedoch weiterhin Hydrolysegas, zwar in geringeren Mengen, jedoch mit einem signifikanten Methananteil (methanhaltiges Hydrolysegas).

Zum Austreiben (Verdrängen) des restlichen methanhaltigen Hydrolysegases aus einem Perkolator (P₂) wird die CO₂-reiche Abluft (CO₂-reiches Hydrolysegas) eines zeitversetzt betriebenen und sich gerade im Abluftbetrieb befindlichen Perkolators (P₁) eingesetzt.

Dafür wird zunächst die Gasklappe (10) des Perkolators (P₂) manuell oder mit Hilfe eines Prozessleitsystems geschlossen. Das Ventil (12) mit Verbindung zur Gasnutzungsanlage (6) des Perkolators (P₂) ist weiterhin geöffnet.

An Perkolator (P₁) wird das Ventil (11) geschlossen (das Ventil (12) ist ebenfalls geschlossen), so dass der Druck innerhalb von Perkolator (P₁) durch die kontinuierlich erfolgende Gasproduktion zu steigen beginnt. Der Drucksensor (17) ermittelt vorzugsweise kontinuierlich den Gassystemdruck innerhalb des Perkolators (P₁). Übersteigt der Gassystemdruck einen zuvor definierten Druck-Schwellenwert, öffnen die Gasklappen (10) der Perkolatoren (P₂ und P₁). Die Perkolatoren (P₂ und P₁) sind somit kopfseitig miteinander verbunden.

Aufgrund des Überdrucks innerhalb des Perkolators (P₁) findet ein gerichteter Gasaustausch aus Perkolator (P₁) in Perkolator (P₂) statt. Über das geöffnete Ventil (12) wird die Gasmischung aus Perkolator (P₂) zur Gasnutzungsanlage (6) geleitet.

Erreicht die Methankonzentration des Gasgemischs aus Hydrolysegas und zugeströmten CO₂-reichem Gas innerhalb des Perkolators (P₂) einen zuvor definierten unteren Grenzwert, wird das Ventil (12) geschlossen. Die Methankonzentration des Gasgemischs in Perkolator (P₂) wird dabei über die Meßeinrichtung (5) ermittelt.

Für den Fall, dass die Methankonzentration den unteren Grenzwert nicht erreicht, kann das Ventil (12) auch manuell geschlossen werden.

Spülung mit Luft: Die Zuluftklappen (10) der Perkolatoren (P₃, P₄) werden geschlossen, sollten sich diese nicht ebenfalls im gleichen Prozessstadium befinden. Das Ventil (11) des Perkolators (P₂) und das Ventil (8) an der Gaszufuhrleitung (3) wird geöffnet, jedoch wird nunmehr über die Gaszufuhrleitung (3) Umgebungsluft eingetragen.

Das Abluftgebläse (13) wird eingeschaltet und dient zum Absaugen des im Perkolator (P₂) enthaltenen Gases. Über die Meßeinrichtung (5) wird die Gaszusammensetzung im Perkolator (P₂) bestimmt. Wenn die durch die Meßeinrichtung (5) ermittelte Methankonzentration- und die Konzentration des inerten Gases einen zuvor definierten jeweiligen Minimalwert (vorzugsweise weniger als 1 Vol.% für Methan, vorzugsweise weniger als 1,5 Vol.-% für CO₂) erreicht hat, wird signalisiert, dass der Perkolator (P₂) geöffnet, entleert und neu beschickt werden kann.

Mit der Neubeschickung beginnt der Zyklus aus Abluftbetrieb, Gasnutzungsbetrieb, Spülung und Öffnung von neuem.

### Beispiel 3: Erfindungsgemäße Anlage zur zweistufigen Biogasgewinnung mit vier Perkolatoren in Parallelschaltung mit zwei Methanreaktoren

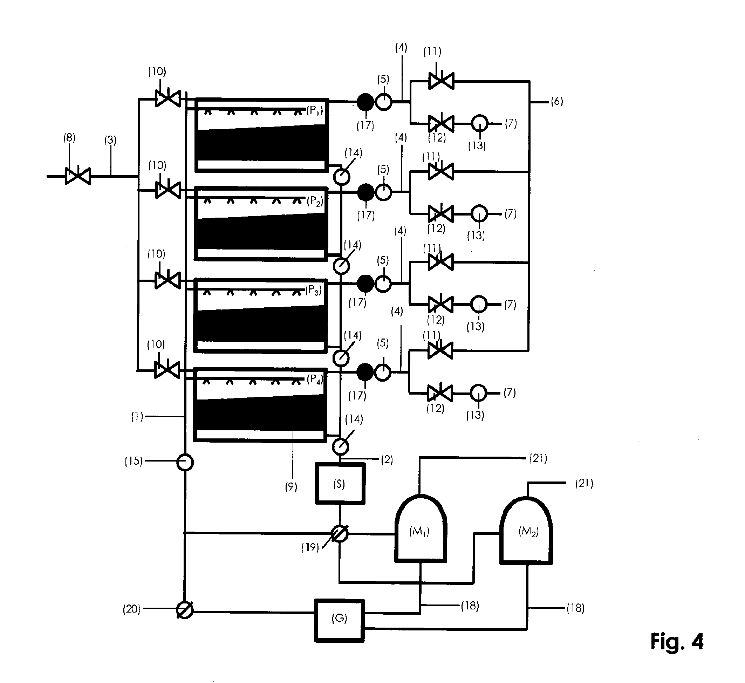

Die Anlage umfasst vier Hydrolysereaktoren (P₁-P₄), in diesem Fall Feststoffperkolatoren, wie in Fig. 4 dargestellt und zwei Methanreaktoren. Die Anlage ist analog zu Beispiel 1 ausgestaltet und wird ebenso betrieben. Der Unterschied zur Anlage aus Beispiel 1 liegt in der Anordnung zweier Methanreaktoren (M₁, M₂). Diese sind dem Hydrolysat-Speicherbehälter (S) nachgeordnet und über deren jeweilige Hydrolysat-Zuleitung mit diesem verbunden. Über das Ventil (19) ist ein Umschalten der Hydrolysatzufuhr in Methanreaktor (M₁) oder Methanreaktor (M₂) möglich. Jeder der Methanreaktoren (M₁, M₂) weist eine Biogasableitung (21) auf. Den Methanreaktoren (M₁, M₂) ist der Gärflüssigkeits-Speicherbehälter (G) über deren Gärflüssigkeit-Ableitungen (18) nachgeordnet.

Im Betrieb wird das Hydrolysat aus dem Hydrolysat-Speicherbehälter (S) in einen der Methanreaktoren geleitet. Die Gärflüssigkeit wird aus den Methanreaktoren (M₁, M₂) in den Gärflüssigkeits-Speicherbehälter (G) überführt.

### Beispiel 4: Erfindungsgemäße Anlage zur zweistufigen Biogasgewinnung mit vier Perkolatoren in Reihenschaltung mit zwei Methanreaktoren

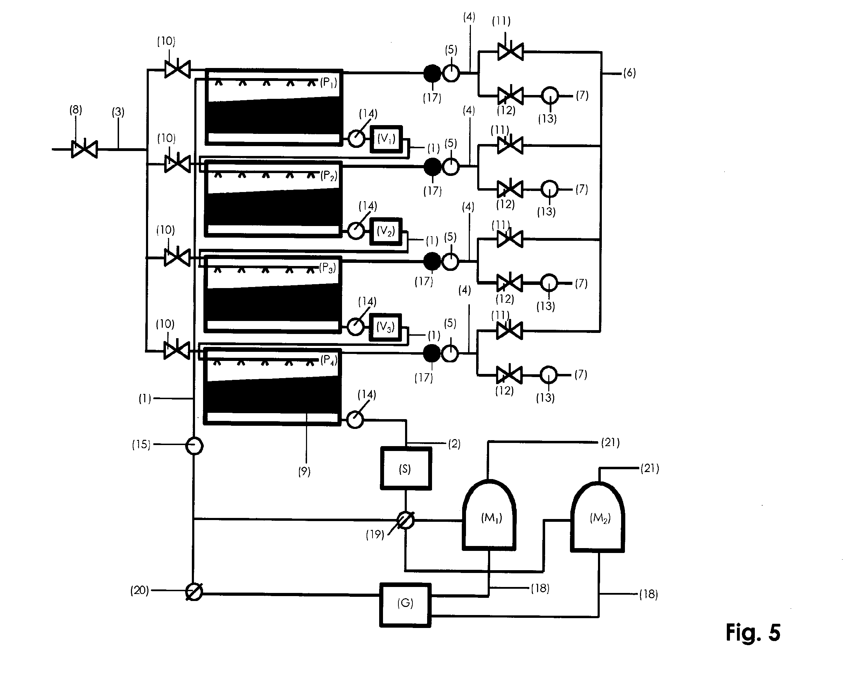

Die Anlage umfasst vier Hydrolysereaktoren (P₁-P₄), in diesem Fall Feststoffperkolatoren, wie in Fig. 5 dargestellt und zwei Methanreaktoren. Die Anlage ist analog zu Beispiel 2 ausgestaltet und wird ebenso betrieben. Der Unterschied zur Anlage aus Beispiel 1 liegt in der Anordnung zweier Methanreaktoren (M₁, M₂). Diese sind dem Hydrolysat-Speicherbehälter (S) nachgeordnet und über deren jeweilige Hydrolysat-Zuleitung mit diesem verbunden. Über das Ventil (19) ist ein Umschalten der Hydrolysatzufuhr in Methanreaktor (M₁) oder Methanreaktor (M₂) möglich. Jeder der Methanreaktoren (M₁, M₂) weist eine Biogasableitung (21) auf. Den Methanreaktoren (M₁, M₂) ist der Gärflüssigkeits-Speicherbehälter (G) über deren Gärflüssigkeit-Ableitungen (18) nachgeordnet.

Im Betrieb wird das Hydrolysat aus dem Hydrolysat-Speicherbehälter (S) in einen der Methanreaktoren geleitet. Die Gärflüssigkeit wird aus den Methanreaktoren (M₁, M₂) in den Gärflüssigkeits-Speicherbehälter (G) überführt.

### Bezugsnummernliste

- (1): Flüssigkeits-Zuleitung
- (2): Hydrolysat-Ableitung
- (3): Gas-Zuleitung
- (4): Gas-Ableitung
- (5): Meßeinrichtung zur Bestimmung der Gasqualität und -menge
- (6): Gasnutzungsanlage
- (7): Abluftbehandlung
- (8): Ventil
- (9): Gitterrost oder Siebboden
- (10): Gasklappe
- (11): Ventil
- (12): Ventil
- (13): Abluftgebläse
- (14): pH-Sensor
- (15): pH-Sensor
- (16): Ventil
- (17): Drucksensor
- (18): Gärflüssigkeits-Ableitung
- (19): Ventil
- (20): Ventil
- (21): Biogasableitung

- (G): Gärflüssigkeits-Speicherbehälter
- (M): Methanreaktor
- (Mₓ): Methanreaktor einer Anlage mit x Methanreaktoren, mit x ≥ 1
- (P): Perkolator
- (Pₙ): Perkolator einer Anlage mit n Perkolatoren, mit n ≥ 1
- (S): Hydrolysat-Speicherbehälter
- (V): Hydrolysat-Vorspeicher
- (Vₙ): Hydrolysat-Vorspeicher einer Anlage mit n Hydrolysat-Vorspeichern, mit n ≥ 1

## Patentansprüche

1. Verfahren zur zwei- oder mehrstufigen Biogasgewinnung durch Hydrolyse fester biogener Stoffe in mindestens zwei zeitlich versetzt betriebenen Perkolatoren, umfassend eine Hydrolyse- und Methanstufe, wobei biogene Stoffe in der Hydrolysestufe hydrolysiert werden und dabei im Perkolator flüssiges Hydrolysat und Hydrolysegas entsteht, wobei zunächst CO₂-reiches Hydrolysegas und anschließend methanhaltiges Hydrolysegas entsteht, und das Hydrolysat aus den Perkolatoren entnommen und gesammelt wird, wobei ein Teil des Hydrolysats der Methanstufe zugeführt wird und der andere Teil des Hydrolysats der Hydrolysestufe zugeführt wird, und wobei das Hydrolysat in der Methanstufe durch methanbildende Mikroorganismen zu Biogas und Gärflüssigkeit umgewandelt wird, und die Gärflüssigkeit aus dem Methanreaktor entnommen und gesammelt wird und ggf. der Hydrolysestufe zugeführt wird, **dadurch gekennzeichnet, dass** die Perkolatoren gasdicht betrieben werden und Hydrolysegas aus den Perkolatoren abgeleitet wird, wobei methanhaltiges Hydrolysegas einer energetischen Nutzung zugeführt wird und CO₂-reiches Hydrolysegas aus einem Perkolator zur Spülung von einem weiteren, zeitlich versetzt betriebenen Perkolator genutzt wird.

2. Verfahren nach Anspruch 1, **dadurch gekennzeichnet, dass** der Betrieb eines Perkolators die wie folgt angeordneten Betriebsstufen umfasst:
a. Beladung eines Perkolators mit den biogenen Stoffen,
b. Abluftbetrieb,
c. Gasnutzungsbetrieb,
d. Spülung des Perkolators mit Abluft aus einem zeitlich versetzt betriebenen Perkolator,
e. Spülung des Perkolators mit Luft,
f. Öffnung des Perkolators,
wobei das Hydrolysegas im Abluftbetrieb als Abluft aus dem Perkolator abgeleitet wird, wobei das Hydrolysegas im Gasnutzungsbetrieb aus dem Perkolator abgeleitet und einer energetischen Nutzung zugeführt wird und wobei das im Perkolator vorliegende Hydrolysegas bei der Spülung in Stufe d. durch Verdrängung mit zugeleiteter Abluft aus einem zeitlich versetzt betriebenen Perkolator entfernt wird.

3. Verfahren nach Anspruch 2, **dadurch gekennzeichnet, dass** der Abluftbetrieb so lange durchgeführt wird, bis die Methankonzentration des im Perkolator befindlichen Gases einen zuvor definierten Grenzwert erreicht und/oder bis der pH-Wert des Hydrolysats einen zuvor definierten Grenzwert erreicht.

4. Verfahren nach Anspruch 3, **dadurch gekennzeichnet, dass** der Gasnutzungsbetrieb so lange durchgeführt wird, bis die Methankonzentration des im Perkolator befindlichen Gases niedriger als der Grenzwert ist.

5. Verfahren nach einem der Ansprüche 2 bis 4, **dadurch gekennzeichnet, dass** die mindestens zwei zeitlich versetzt betriebenen Perkolatoren so betrieben werden, dass sich mindestens ein Perkolator im Abluftbetrieb befindet.

6. Verfahren nach Anspruch 5, **dadurch gekennzeichnet, dass** vor der Spülung eines Perkolators ein zu dem zu spülenden Perkolator zeitversetzt betriebener Perkolator, welcher im Abluftbetrieb betrieben wird, verschlossen wird, so dass ein Überdruck in diesem Perkolator entsteht und nach Erreichen eines Druck-Schwellenwertes anschließend die Abluft aus dem zeitversetzt betriebenen Perkolator in den zu spülenden Perkolator geleitet wird.

7. Anlage zur zwei- oder mehrstufigen Biogasgewinnung, umfassend mindestens zwei Perkolatoren (P₁, P₂), insbesondere Feststoffperkolatoren, welche jeweils eine Hydrolysat-Ableitung (2) und jeweils eine Flüssigkeitszuleitung (1) aufweisen, wobei die Hydrolysat-Ableitung (2) über mindestens einen Hydrolysat-Speicherbehälter (S) mit mindestens einem Methanreaktor (M) verbunden ist, und dem mindestens einen Methanreaktor (M) über eine Gärflüssigkeits-Ableitung (18) mindestens ein Gärflüssigkeits-Speicherbehälter (G) nachgeordnet ist, wobei Hydrolysat-Speicherbehälter (S) und Gärflüssigkeits-Speicherbehälter (G) mit der jeweiligen Flüssigkeitszuleitung (1) des Perkolators (P₁, P₂) verbunden sind, **dadurch gekennzeichnet, dass** die mindestens zwei Perkolatoren (P₁, P₂) gasdicht ausgestaltet sind und jeweils mindestens eine jeweils verschließbare Gas-Zuleitung (3) und Gas-Ableitung (4) umfassen, und jeder der Perkolatoren (P₁, P₂) mit einem Methansensor verbunden ist, welcher zur Bestimmung des Methangehalts des im jeweiligen Perkolator (P₁, P₂) enthaltenen Gases dient und mit einem pH-Sensor (14) verbunden ist, welcher zur Bestimmung des pH-Werts der im jeweiligen Perkolator (P₁, P₂) enthaltenen Flüssigkeit dient, und die Gas-Zuleitung (3) des jeweiligen Perkolators (P₁, P₂) so ausgestaltet ist, dass ein Umschalten auf Luftzufuhr, auf Zufuhr von Gas aus einem weiteren Perkolator oder ein Verschluss der Gas-Zuleitung möglich ist, wobei die mindestens zwei Perkolatoren (P₁, P₂) untereinander über deren Gas-Zuleitungen (3) verbunden sind, so dass bei zeitversetztem Betrieb die Einleitung von CO₂-reichem Hydrolysegas von einem Perkolator (P₁) im Abluftbetrieb in einen zu spülenden Perkolator (P₂) erfolgen kann.

8. Anlage nach Anspruch 7, **dadurch gekennzeichnet, dass** jeder der Perkolatoren (P₁, P₂) jeweils eine verschließbare Gas-Ableitung (4) umfassen, wobei die Gas-Ableitung (4) so ausgestaltet ist, dass ein Umschalten auf Gasableitung zu einer Gasnutzungsanlage, Gasableitung in die Atmosphäre oder ein Verschluss der Gas-Ableitung möglich ist.

9. Anlage nach Anspruch 7 oder 8, **dadurch gekennzeichnet, dass** an den Perkolatoren (P₁, P₂) jeweils ein Drucksensor (18) angeordnet ist, welcher zur Druckmessung im Perkolator (P₁, P₂) dient.

## Claims

1. A method for obtaining biogas in two or more stages by hydrolysis of solid biogenic material in at least two percolators operated at offset times, comprising a hydrolysis stage and a methane stage, wherein biogenic material is hydrolysed in the hydrolysis stage, whereby in the percolator liquid hydrolysate and hydrolysis gas is formed, whereby at first CO₂-rich hydrolysis gas and afterwards methane-containing hydrolysis gas is formed, and whereby the hydrolysate is removed from the percolators and collected, whereby one part of the hydrolysate is fed into the methane stage, and the other part of the hydrolysate is fed into the hydrolysis stage, whereby the hydrolysate in the methane stage is converted into biogas and fermentation liquid by methane-forming microorganisms, and the fermentation liquid is removed from the methane reactor and collected, and optionally fed into the hydrolysis stage, **characterized in that** the percolators are operated in a gas-tight manner and that hydrolysis gas is drawn off from the percolators, whereby methane-containing hydrolysis gas is used for energy recovery, and CO₂-rich hydrolysis gas from one percolator is used for purging another percolator, which is operated at offset times.

2. A method according to claim 1, **characterized in that** the operation of a percolator comprises the steps of operation in the following order:
a. Loading a percolator with the biogenic material,
b. Exhaust air mode,
c. Gas utilising mode,
d. Purging the percolator with exhaust air from another percolator that is operated at offset times,
e. Purging the percolator with air,
f. Opening the percolator,
whereby in Exhaust air mode the hydrolysis gas is drawn off as exhaust air from the percolator, whereby in Gas utilising mode the hydrolysis gas is drawn off from the percolator and is used for energy recovery, and whereby the hydrolysis gas present inside the percolator during Purging in step d. is removed by expelling it by means of fed-in exhaust air from a percolator that is operated at offset times.

3. A method according to claim 2, **characterized in that** the Exhaust air mode is continued until the methane concentration of the gas present inside the percolator reaches a previously defined limit value and/or until the pH value of the hydrolysate reaches a previously defined limit value.

4. A method according to claim 3, **characterized in that** the Gas utilising mode is continued until the methane concentration of the gas present inside the percolator is lower than the limit value.

5. A method according to one claims 2 to 4, **characterized in that** the at least two percolators operated at offset times are operated in such a manner that at least one percolator is operated in Exhaust air mode.

6. A method according to claim 5, **characterized in that** before Purging one percolator another percolator that is operated at offset times in relation to the percolator to be purged and which is operated in Exhaust air mode, is closed, so that overpressure arises in this percolator, and after reaching a threshold value of the pressure, the exhaust air is subsequently transported from the percolator operated at offset times into the percolator to be purged.

7. A system for obtaining biogas in two or more stages, comprising at least two percolators (P₁, P₂), preferably solids percolators, each of which comprises one hydrolysate discharge pipe (2) and one liquids feed inlet (1), whereby the hydrolysate discharge pipe (2) is connected to at least one methane reactor (M) via at least one storage tank for hydrolysate (S), and whereby downstream to the at least one methane reactor at least one storage tank for fermentation liquid (G) is arranged, connected via a discharge pipe for fermentation liquid (18), whereby the storage tank for hydrolysate (S) and the storage tank for fermentation liquid (G) are connected to the respective liquids feed inlet (1) of the percolator (P₁, P₂), **characterized in that** the at least two percolators (P₁, P₂) are designed to be gas-tight and comprise each at least one closable gas supply pipe (3) and at least one closable gas discharge pipe (4), and each of the percolators (P₁, P₂) is connected to a methane sensor which serves to measure the methane content of the gas present in the respective percolator (P₁, P₂) and which is connected to a pH sensor (14), which serves to measure the pH value of the liquid present in the respective percolator (P₁, P₂), and wherein the gas supply pipe (3) of the respective percolator (P₁, P₂) is designed in such a manner that it is possible to switch to air supply, to gas supply from another percolator, or to close the gas supply pipe, whereby the at least two percolators (P₁, P₂) are connected to each other via their gas supply pipes (3), so that during operation at offset times the feed-in of CO₂-rich hydrolysis gas from one percolator (P₁) in exhaust air mode into a percolator to be purged (P₂) can take place.

8. A system according to claim 7, **characterized in that** each of the percolators (P₁, P₂) comprises a closable gas discharge pipe (4), whereby the gas discharge pipe (4) is designed to allow either switching to gas discharge into a system for energy utilisation, or discharge of the off-gas into the atmosphere, or closure of the gas discharge pipe.

9. A system according to claim 7 or 8, **characterized in that** a pressure sensor (17) is arranged at each of the percolators (P₁, P₂), which serves to measure the pressure inside the percolator (P₁, P₂).

## Revendications

1. Procédé de production de biogaz en deux ou plusieurs étapes par hydrolyse de substances biogènes solides dans au moins deux percolateurs fonctionnant de manière décalée dans le temps, comprenant une étape d'hydrolyse et une étape de méthanisation, des substances biogènes étant hydrolysées dans l'étape d'hydrolyse et un hydrolysat liquide et un gaz d'hydrolyse étant produits à cette occasion dans le percolateur, un gaz d'hydrolyse riche en CO₂ étant produit d'abord et un gaz d'hydrolyse contenant du méthane ensuite, l'hydrolysat étant extrait des percolateurs et collecté, une partie de l'hydrolysat étant amenée à l'étape de méthanisation et l'autre partie de l'hydrolysat à l'étape d'hydrolyse, et l'hydrolysat étant transformé dans l'étape de méthanisation en biogaz et en liquide de fermentation par des micro-organismes méthanogènes, et le liquide de fermentation étant prélevé du réacteur de méthanisation et collecté puis, le cas échéant, amené à l'étape d'hydrolyse, **caractérisé en ce que** les percolateurs fonctionnent de manière étanche aux gaz et le gaz d'hydrolyse est évacué des percolateurs, le gaz d'hydrolyse contenant du méthane étant amené à une utilisation énergétique et le gaz d'hydrolyse riche en CO₂ provenant d'un percolateur étant utilisé pour rincer un autre percolateur fonctionnant de manière décalée dans le temps.

2. Procédé selon la revendication 1, **caractérisé en ce que** le fonctionnement d'un percolateur comprend les étapes ordonnées comme suit :
a. chargement d'un percolateur avec les substances biogènes,
b. fonctionnement en mode air d'évacuation,
c. fonctionnement en mode utilisation du gaz,
d. rinçage du percolateur avec de l'air d'évacuation provenant d'un percolateur fonctionnant de manière décalée dans le temps,
e. rinçage du percolateur avec de l'air,
f. ouverture du percolateur,
le gaz d'hydrolyse dans le fonctionnement en mode air d'évacuation étant évacué du percolateur sous forme d'air d'évacuation, le gaz d'hydrolyse dans le fonctionnement en mode utilisation du gaz étant évacué du percolateur et amené à une utilisation énergétique et le gaz d'hydrolyse présent dans le percolateur étant éliminé lors du rinçage à l'étape d. par refoulement avec de l'air d'évacuation provenant d'un percolateur fonctionnant de manière décalée dans le temps.

3. Procédé selon la revendication 2, **caractérisé en ce que** le fonctionnement en mode air d'évacuation est appliqué jusqu'à ce que la concentration en méthane du gaz se trouvant dans le percolateur atteigne une valeur limite préalablement définie et/ou jusqu'à ce que le pH de l'hydrolysat atteigne une valeur limite préalablement définie.

4. Procédé selon la revendication 3, **caractérisé en ce que** le fonctionnement en mode utilisation du gaz est appliqué jusqu'à ce que la concentration en méthane du gaz se trouvant dans le percolateur soit inférieure à la valeur limite.

5. Procédé selon une des revendications 2 à 4, **caractérisé en ce que** lesdits au moins deux percolateurs fonctionnant de manière décalée dans le temps fonctionnent de façon qu'au moins un percolateur se trouve en mode air d'évacuation.

6. Procédé selon la revendication 5, **caractérisé en ce qu'**avant le rinçage d'un percolateur, un percolateur fonctionnant de manière décalée dans le temps par rapport au percolateur à rincer, lequel fonctionne en mode air d'évacuation, est fermé, de sorte qu'il apparaît une surpression dans ce percolateur et, après atteinte d'une valeur seuil de pression, l'air d'évacuation provenant du percolateur fonctionnant de manière décalée dans le temps est conduit dans le percolateur à rincer.

7. Installation de production de biogaz en deux ou plusieurs étapes, comprenant au moins deux percolateurs (P₁, P₂), en particulier des percolateurs de matières solides, lesquels présentent chacun une conduite d'évacuation d'hydrolysat (2) et une conduite d'amenée de liquide (1), la conduite d'évacuation d'hydrolysat (2) étant reliée à au moins un réacteur de méthanisation (M) via au moins un réservoir de stockage d'hydrolysat (S), et au moins un réservoir de stockage de liquide de fermentation (G) étant placé à la suite dudit au moins un réacteur de méthanisation (M) via une conduite d'évacuation de liquide de fermentation (18), le réservoir de stockage d'hydrolysat (S) et le réservoir de stockage de liquide de fermentation (G) étant reliés à la conduite d'amenée de liquide (1) respective du percolateur (P₁, P₂), **caractérisée en ce que** lesdits au moins deux percolateurs (P₁, P₂) sont conçus de manière étanche aux gaz et comprennent chacun au moins une conduite d'amenée de gaz (3) et une conduite d'évacuation de gaz (4) pouvant chacune être fermée, et chacun des percolateurs (P₁, P₂) est relié à un capteur de méthane, lequel sert à déterminer la teneur en méthane du gaz contenu dans le percolateur (P₁, P₂) respectif et à un capteur de pH (14), lequel sert à déterminer le pH du liquide contenu dans le percolateur (P₁, P₂) respectif, et la conduite d'amenée de gaz (3) du percolateur (P₁, P₂) respectif est conçue de façon qu'une commutation sur amenée d'air, sur amenée de gaz d'un autre percolateur ou qu'une fermeture de la conduite d'amenée de gaz soit possible, lesdits au moins deux percolateurs (P₁, P₂) étant reliés entre eux par leurs conduites d'amenée de gaz (3) de façon qu'en cas de fonctionnement décalé dans le temps, du gaz d'hydrolyse riche en CO₂ d'un percolateur (P₁) fonctionnant en mode air d'évacuation puisse être introduit dans un percolateur (P₂) à rincer.

8. Installation selon la revendication 7, **caractérisée en ce que** chacun des percolateurs (P₁, P₂) comprend une conduite d'évacuation de gaz (4) pouvant être fermée, la conduite d'évacuation de gaz (4) étant conçue de façon qu'une commutation sur évacuation de gaz vers une installation d'utilisation du gaz, évacuation de gaz dans l'atmosphère ou qu'une fermeture de la conduite d'évacuation de gaz soit possible.

9. Installation selon la revendication 7 ou 8, **caractérisée en ce qu'**un capteur de pression (18) est disposé sur chacun des percolateurs (P₁, P₂), lequel sert à mesurer la pression dans le percolateur (P₁, P₂).
